(19) Europäisches Patentamt
European Patent Office
Office européen des brevets

(11) **EP 4 057 229 A1**

(12) **EUROPÄISCHE PATENTANMELDUNG**

(43) Veröffentlichungstag:
**14.09.2022 Patentblatt 2022/37**

(51) Internationale Patentklassifikation (IPC):
***G06T 11/00*** (2006.01)

(21) Anmeldenummer: **21162349.1**

(52) Gemeinsame Patentklassifikation (CPC):
**G06T 11/005;** G06T 2211/408

(22) Anmeldetag: **12.03.2021**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Fraunhofer-Gesellschaft zur Förderung der angewandten Forschung e.V. 80686 München (DE)**

(72) Erfinder:
• **FIRSCHING, Markus**
 **91058 Erlangen (DE)**
• **ENNEN, Alexander**
 **91058 Erlangen (DE)**
• **SPEIER, Christoph**
 **91058 Erlangen (DE)**

(74) Vertreter: **Burger, Markus et al Schoppe, Zimmermann, Stöckeler Zinkler, Schenk & Partner mbB Patentanwälte Radlkoferstraße 2 81373 München (DE)**

(54) **VERARBEITUNGSVORRICHTUNG ZUM ERHALTEN EINER LÜCKENFÜLLER-SINOGRAMMINFORMATION, COMPUTERTOMOGRAPH, VERFAHREN UND COMPUTERPROGRAMM**

(57)    1. Verarbeitungsvorrichtung (100) zum Erhalten einer Lückenfüller-Sinogramminformation
wobei die Verarbeitungsvorrichtung (100) ausgelegt ist, um eine erste Sinogramminformation, die einem ersten spektralen Parameter zugeordnet ist, und
eine zweite Sinogramminformation, die einem zweiten spektralen Parameter zugeordnet ist, zu erhalten, und
wobei die Verarbeitungsvorrichtung (100) ausgelegt ist, um die Lückenfüller-Sinogramminformation, die dem zweiten spektralen Parameter zugeordnet ist, und die die eine Lücke (520, 620, 720) in der zweiten Sinogramminformation füllt, basierend auf der ersten Sinogramminformation und der zweiten Sinogramminformation zu erhalten.

Ein Computerprogramm, ein Verfahren und eine Computer-Implementierung werden ebenfalls beschrieben.

Fig. 1

**Beschreibung**

**Gebiet der Erfindung**

Ausführungsformen gemäß der Erfindung beziehen sich auf eine Verarbeitungsvorrichtung zum Erhalten einer Lückenfüller-Sinogramminformation.

Einige Ausführungsformen gemäß der Erfindung beziehen sich auf einen Computertomographen umfassend eine Verarbeitungsvorrichtung zum Erhalten einer Lückenfüller-Sinogramminformation.

Einige Ausführungsformen gemäß der Erfindung beziehen sich auf ein Verfahren zum Erhalten einer Lückenfüller-Sinogramminformation.

Weitere Ausführungsformen gemäß der Erfindung beziehen sich auf ein Computerprogramm.

Ausführungsbeispiele der Erfindung beziehen sich auf eine Verarbeitungsvorrichtung zur Vervollständigung unvollständiger Dual-Energy CT-Daten mithilfe tiefer neuronaler Netze zur Verringerung der Aufnahmezeit und/oder Reduktion der Dosis, ein Verfahren und ein Computerprogramm.

**Hintergrund der Erfindung**

Röntgen-Computer-Tomographie (CT) ist ein in der Medizin und auch in der industriellen Anwendung weit verbreitete bildgebende Methode. Hierbei wird eine größere Anzahl (typischerweise hunderte bis tausende) von Durchstrahlungsbildern in verschiedenen Projektionsrichtungen aufgenommen. Die Darstellung einer Ortsdimension (eine Reihe von Detektorpixeln senkrecht zur Drehachse) und der Winkeldimension (Rotationswinkel zwischen Objekt und Abbildungssystem) wird Sinogramm genannt. Anschließend kann aus diesem Sinogramm ein tomographisches Schnittbild rekonstruiert werden [1]. Einen Detektor mit mehreren Detektionszeilen vorausgesetzt, ist es ebenso möglich, mehrere Schichten aus den Pixelreihen senkrecht bezüglich der Drehachse (also beispielsweise mehrere Sinogramme) gleichzeitig zu rekonstruieren [2]. Die Datenstruktur, die die Projektionsdaten eines solchen mehrzeiligen Detektors beinhaltet (also das Sinogramm im weiteren Sinne), ist also dreidimensional. Im Folgenden wird die Notation verwendet, in der die erste Dimension ("x") die Pixelnummer entlang der Zeile beschreibt, die zweite Dimension ("y") die Zeilennummer und die dritte Dimension ("z" oder "$\alpha$") den Winkelschritt. Für gewöhnlich ist diese erste Dimension eines solchen erweiterten Sinogramms im Wesentlichen senkrecht zur Drehachse, während die zweite Dimension im Wesentlichen parallel zur Drehachse ist.

Einerseits erfordert nun die Aufnahme solcher Röntgenprojektionen eine gewisse Zeit und ist mit einer gewissen (potentiell schädlichen) Dosisbelastung [3] für den Patienten bzw. dem Untersuchungsobjekt verbunden. Beides ist aus offensichtlichen Gründen möglichst klein zu halten. In der medizinischen Anwendung steht die Verringerung der Dosis im Vordergrund, während in technisch-industriellen Anwendungen mit unbelebten Untersuchungsobjekten die Aufnahmedauer (und damit der erzielbare Arbeitstakt) im Vordergrund steht. Andererseits erfordert eine qualitativ hochwertige CT Aufnahme hinreichend große Anzahl dieser Projektionsaufnahmen. Eine zu kurze Belichtungszeit und/oder eine zu geringe Anzahl führt in der Regel zu unschärferen und/oder verrauschteren CT-Bildern.

Die Dual-Energy-Methode (oder Zwei-Energien Methode) ist in der Computer Tomographie ("Dual-Energy-CT", Zwei-Energie-Computertomographie, Mehrenergie-Röntgentechnik oder Zwei-Spektren-CT) eine bekannte Technik, um Strahlaufhärtungsartefakte zu reduzieren [4, 5, 6]. Des Weiteren ermöglicht Dual-Energy-CT nicht nur die Korrektur von Strahlaufhärtungsartefakten in der CT, sondern auch zusätzliche Materialinformation über das untersuchte Objekt zu erlangen [7, 8].

Für eine Dual-Energy-CT sind zwei CT-Datensätze mit unterschiedlichen spektralen Parametern nötig, diese werden vereinfacht "low energy" (LE, niedrige Energie) und "high energy" (HE, hohe Energie) genannt. Anpassung der spektralen Parameter können z.B. eine Änderung der Beschleunigungsspannung der Röntgenröhre und/oder die Verwendung zusätzlicher Vorfilter sein.

Um Dual-Energy-Daten aufzunehmen, ist es notwendig, entweder nacheinander zwei Scans bei unterschiedlichen spektralen Parametern aufzunehmen oder mit zwei Röntgenquellen und zwei Detektoren zwei Scans gleichzeitig aufzunehmen. In beiden Fällen führt dies ungefähr zu einer Verdopplung der Dosis, im ersten Fall zusätzlich zur Verdopplung der Aufnahmezeit, im zweiten Fall zusätzlich zu doppelten Kosten für die Röntgen-Hardware und insgesamt erhöhtem konstruktivem Aufwand.

Eine von mehreren möglichen Implementierungen von Dual-Energy-CT besteht normalerweise aus den folgenden Schritten: Zunächst werden die Projektionsdaten für eine Standard-CT aufgenommen. Anschließen wird noch ein zweiter CT-Projektionsdatensatz mit angepassten spektralen Parametern (z.B. Veränderung von Beschleunigungsspannung und/oder Vorfilter) aufgenommen. Die Aufnahmezeit und die Dosis erhöht sich also. Zusätzliche Hardware ist nicht notwendig. Diese beiden Datensätze werden mit Dual-Energy-Methoden (Zwei-Energie-Methoden) weiterverarbeitet und anschließend erfolgt die CT-Rekonstruktion der Schnittbilder. Alternativ kann der zweite Datensatz mit den anderen spektralen Parametern auch gleichzeitig mit einem zweiten Satz an Röntgenkomponenten (mindestens bestehend aus Röntgenquelle und Detektor) aufgenommen werden. Auf die darauffolgenden Schritte der Datenverarbeitung hat dies keinen Einfluss.

Im Folgenden werden einige Nachteile von Dual-Energy Methoden beschrieben:
Einerseits erfordert nun die Aufnahme solcher Röntgenprojektionen eine gewisse Zeit und ist mit einer gewissen (potentiell schädlichen) Dosisbelastung für den Patienten bzw. dem Untersuchungsobjekt verbunden. Beides ist aus (offensichtlichen) Gründen möglichst klein zu halten. In der medizinischen Anwendung steht die Verringerung der Dosis im Vordergrund, während in technisch-industriellen Anwendungen mit unbelebten Untersuchungsobjekten die Aufnahmedauer (und damit der erzielbare Arbeitstakt) im Vordergrund steht. Andererseits erfordert eine qualitativ hochwertige CT Aufnahme eine hinreichend große Anzahl dieser Projektionsaufnahmen. Eine zu geringe Anzahl führt in der Regel zu unschärferen und/oder verrauschteren CT-Bildern.

Dual-Energy-CT ist nach dem Stand der Technik selbst bei verschiedenen Umsetzungen also mit einem Nachteil versehen: erhöhte Dosis und erhöhte Aufnahmedauer oder erhöhte Dosis und erhöhter apparativer Aufwand. Dies führt dazu, dass Dual-Energy-CT nur in relativ wenigen, speziellen Anwendungen ökonomisch eingesetzt werden kann, obwohl der Informationsgewinn in weit mehr Fällen signifikant sein kann [9].

Dual-Energy-CT stellt eine gute Möglichkeit dar, den Kontrast zwischen Materialien im 3D Volumen zu erhöhen oder Artefakte im 3D Volumen zu korrigieren. Für eine Dual-Energy-CT Messung müssen jedoch (zumindest in manchen Fällen) mindestens zwei vollständige CT Messungen des gleichen Objekts bei unterschiedlichen spektralen Parametern durchgeführt werden. Dies erfolgt entweder durch die Durchführung einer zweiten Messung oder einem Kreuzaufbau, welcher aus 2 CT Systemen besteht. Problematisch hierbei ist die Erhöhung der Messzeit (Faktor 2) oder die Erhöhung der Anlagenkosten (zweites CT-System). Einerseits werden Deep Learning Ansätze zunehmend verwendet, um Verbesserungen in der Bildverarbeitung auch von Dual Energy Röntgendaten zu erreichen [10]. Andererseits sind Methoden zur künstlichen Erzeugung von Röntgenprojektionsaufnahmen mithilfe generativer neuronaler Netze bekannt [11].

Aus Sicht der CT-Rekonstruktion stellen fehlende Aufnahmen von bestimmten Projektionswinkeln Lücken im Sinogramm dar. Es sind Methoden zur CT-Rekonstruktion lückenbehafteter Sinogramme bekannt, die auf Deep Learning Methoden beruhen und beispielsweise GANs zur Rekonstruktion verwenden [12] oder Methoden, die unvollständige Sinogramme mit Hilfe von GANs erweitern [13, 14, 15].

Des Weiteren ist eine Dual Energy Methode bekannt, die im Falle eines beschränkten Sichtfelds in einer der beiden Bildgebungsketten ("limited field of view") im rekonstruierten Voxelvolumen (also nicht auf Ebene der Projektionen bzw. Sinogramme) die fehlenden Bereiche mit Hilfe der strukturellen Information aus der anderen, vollständigen Bildgebungskette schätzt, um einen vollständigen Datensatz zu erhalten [16]. Diese Methode geht von einem Dual source System aus, bei dem die HE Bildgebungskomponente ein reduziertes FoV (Field of View, "Sichtfeld") hat. Diese abgeschnittenen HE Projektionen werden mit den reskalierten Daten aus dem LE System hilfsweise vervollständigt, um Rekonstruktionsartefakte zu vermeiden. Das funktioniert bei einem bekannten Untersuchungsobjekt wie dem menschlichen Körper hinreichend gut um besagte Bildfehler in der Rekonstruktion zu vermeiden. Damit ist aber in diesem Bereich keine Dual Energy Auswertung möglich. Das deep learning System aber arbeitet auf den Voxeldatensätzen und erzeugt die fehlenden Bereiche des limitierten FoV mit Hilfe von deep learning Methoden.

Es besteht daher ein Wunsch, die voranstehend beschriebenen Nachteile bei Dual-Energy-CT zu beheben oder zumindest teilweise zu beheben. Zudem besteht ein Wunsch, eine Verarbeitungsvorrichtung zu schaffen, die (beispielsweise auf eine einfache und kostengünstige Art und Weise) eine geringere Strahlendosis und/oder eine geringere Messzeit und damit geringere Kosten gewährleistet.

Insgesamt besteht der Wunsch, ein Konzept zu schaffen, das einen verbesserten Kompromiss zwischen Bildqualität, Strahlenbelastung, Messzeit und Kosten mit sich bringt.

**Zusammenfassung der Erfindung**

Voranstehende Aufgaben werden durch die Patentansprüche zumindest teilweise gelöst.

Demnach werden die Aufgaben durch eine Verarbeitungsvorrichtung zum Erhalten einer Lückenfüller-Sinogramminformation mit den Merkmalen des unabhängigen Anspruchs 1, und durch einen Computertomographen nach mit den Merkmalen des nebengeordneten Anspruchs 12 gelöst, und ferner durch ein Verfahren zum Erhalten einer Lückenfüller-Sinogramminformation und ein Computerprogramm gelöst.

Weitere Merkmale und Details der Erfindung ergeben sich aus den Unteransprüchen, der Beschreibung und den Zeichnungen. Dabei gelten Merkmale und Details, die im Zusammenhang mit der erfindungsgemäßen Verarbeitungsvorrichtung beschreiben sind, selbstverständlich auch im Zusammenhang mit dem erfindungsgemäßen Computertomographen und jeweils umgekehrt, sodass bezüglich der Offenbarung zu den einzelnen Erfindungsaspekten stets wechselseitig Bezug genommen wird beziehungsweise werden kann.

In anderen Worten, die hierin offenbarten Verfahren können optional um alle Merkmale, Funktionalitäten und Details ergänzt werden, die hierin im Bezug auf die Vorrichtungen offenbart sind, und zwar sowohl einzeln als auch in Kombination.

Die hierin offenbarten Vorrichtungen können optional um alle Merkmale, Funktionalitäten und Details ergänzt werden, die hierin im Bezug auf die Verfahren offenbart sind, und zwar sowohl einzeln als auch in Kombination.

Eine Ausführungsform gemäß der Erfindung stellt eine Verarbeitungsvorrichtung zum Erhalten einer Lückenfüller-Sinogramminformation bereit. Die Verarbeitungsvorrichtung ist dabei ausgelegt, eine erste Sinogramminformation, die einem ersten spektralen Parameter zugeordnet ist zu erhalten. Die Verarbeitungsvorrichtung ist weiter dazu ausgelegt, eine zweite Sinogramminformation, die einem zweiten spektralen Parameter zugeordnet ist, der sich vom ersten spektralen Parameter unterscheidet, zu erhalten. Die Verarbeitungsvorrichtung ist ferner dazu ausgelegt, die Lückenfüller-Sinogramminformation, die dem zweiten spektralen Parameter zugeordnet ist, und die eine Lücke in der zweiten Sinogramminformation füllt, basierend auf der ersten Sinogramminformation und der zweiten Sinogramminformation zu erhalten. Erhalten einer Sinogramminformation kann hierin auch gleichbedeutend mit empfangen oder bestimmen einer Sinogramminformation sein.

Diese Ausführungsform basiert auf der Erkenntnis, dass eine Lücke einer zweiten Sinogramminformation mit den Informationen, die in der erhaltenen (z.B. empfangenen) ersten Sinogramminformation bereits enthalten sind, unter Verwendung der ersten und der zweiten Sinogramminformation zum Erhalten einer Lückenfüller-Sinogramminformation, gefüllt werden kann. Die Vorteile eines Dual-Energy-CT können dadurch (zumindest im Wesentlichen) erhalten bleiben, wie beispielsweise die Korrektur von Strahlaufhärtungsartefakten oder das Erhalten von zusätzlichen Materialinformationen, wie beispielsweise die Möglichkeit verschiedene Materialien zu unterscheiden, selbst wenn die zweite Sinogramminformation Lücken aufweist (beispielsweise nicht für alle Winkelwerte aufgenommen wurde). Entsprechend kann ein zu untersuchendes Objekt weniger Röntgenstrahlung ausgesetzt werden, weil eine zweite Sinogramminformation Lücken enthalten kann (als beispielsweise bei weniger Winkelwerten aufgenommen sein kann), die über die erste Sinogramminformation und die zweite Sinogramminformation durch eine erfindungsgemäße Verarbeitungsvorrichtung beispielsweise (zumindest näherungsweise) rekonstruiert werden können. Alternativ oder ergänzend kann eine erfindungsgemäße Verarbeitungsvorrichtung kostengünstiger betrieben werden, weil beispielsweise weniger Zeit benötigt wird, um eine lückenbehaftete zweite Sinogramminformation zu erzeugen oder zu erhalten, im Gegensatz zu einer vollständigen zweiten Sinogramminformation. Mit dem Erzeugen der Lückenfüller-Sinogramminformation bleiben die Vorteile einer Dual-Energy CT (zumindest im Wesentlichen) erhalten. Anstatt den zweiten Datensatz vollständig aufzunehmen, kann die zweite CT-Messung vorteilhafterweise mit deutlich weniger Winkelschritten durchgeführt werden. Die zum Vergleich (bzw. im Vergleich) mit der ersten Messung fehlenden Winkelschritte werden durch eine erfindungsgemäße Verarbeitungsvorrichtung berechnet/ermittelt bzw. geschätzt. Somit kann es möglich sein, mit einer wesentlich geringeren zusätzlichen Messzeit Dual-Energy-CT Datensätze zu generieren, welche zur Artefaktreduktion oder Kontrastanhebung verwendet werden können. Vorteile der Methode sind verringerte Kosten (Messzeit), und/oder verringerte Dosen und/oder geringere Anlagenkosten (kein Kreuzaufbau erforderlich). Die Merkmale bzw. die Lösung ermöglicht die Vorteile von DE-CT auszunutzen, ohne die - normalerweise doppelte - Messzeit in Kauf nehmen zu müssen. Der Vorteil besteht in der Kostenersparnis und/oder Dosisminimierung.

Sinogramminformationen beziehen sich beispielsweise auf die Informationen, die bei einer Projektion im CT mit Hilfe eines Detektors gewonnen werden können. Sinogramminformation kann beispielsweise weitere Daten umfassen, wie beispielsweise Zeitstempel, Detektorinformation, Winkelschritt-Information oder ähnliche Information, die mit der Projektion oder der Detektion zusammenhängen. Sinogramminformation kann sich beispielsweise auf Sinogrammpixel, -zeilen (z.B. Sinogrammzeilen oder Sinogrammpixelzeilen) oder Sinogrammpatches (z.B. Projektionspatches (Patches räumlich benachbarter Pixel) oder Sinogramm-Bildpunktgruppen) beziehen und/oder den mit den Sinogrammpixeln, - zeilen oder -patches verbundenen Informationsgehalt, wie beispielsweise ein oder mehrere Grauwerte. Unter einem Sinogramm versteht man typischerweise die 2-D Darstellung der Pixelwerte einer Detektorzeile entlang der Projektionswinkel. Im Fall eines Flächendetektors hat man nun keine einzelne Zeile, sondern viele Zeilen. Das "erweiterte" Sinogramm hat jetzt nicht mehr zwei Dimensionen, sondern drei; also zwei räumliche Dimensionen (x- und y-Richtung in der Detektionsebene, sowie den Winkelschritt als dritte Dimension). Sinogramminformation wie sie hierin verwendet wird, kann sich sowohl auf ein Sinogramm in zwei, als auch in drei Dimensionen beziehen.

Lückenfüller-Sinogramminformation, wie hierin verwendet, kann sich beispielsweise auf ein synthetisiertes Sinogrammpixel beziehen und/oder den mit dem synthetisierten Sinogrammpixel verbundenen (assoziierten) Informationsgehalt. Lückenfüller-Sinogramminformation kann beispielsweise weitere Informationen umfassen, wie beispielsweise einen Zeitstempel oder dem Pixel zugeordnete Koordinaten. Lückenfüller-Sinogramminformation kann sich beispielsweise auf eine synthetisierte Sinogrammzeile beziehen und/oder den mit der synthetisierten Sinogrammzeile verbundenen Informationsgehalt. Lückenfüller-Sinogramminformation kann sich beispielsweise auf einen synthetisierten Sinogrammpatch beziehen und/oder den mit dem synthetisierten Sinogrammpatch verbundenen Informationsgehalt. Gemäß Ausführungsformen ist die Verarbeitungsvorrichtung dazu ausgelegt, Lückenfüller-Sinogramminformation zu erzeugen, unter Verwendung von erste Sinogramminformation und zweiter Sinogramminformation.

Gemäß einer bevorzugten Ausführungsform der Erfindung entspricht die Lücke einem oder mehreren Pixeln. Die Lücke kann beispielsweise einem oder mehreren Pixelwerten entsprechen. Ein Pixel kann beispielsweise einem Grauwert im betreffenden Projektionsbild entsprechen. Ein Pixelwert kann beispielsweise einer von dem Pixel des Detektors gewonnenen Information entsprechen. Eine Lücke, die mehrere Pixel oder Pixelwerte umfasst, kann beispielsweise einer Pixelzeile entsprechen oder einer Pixelspalte. Eine Lücke, die mehrere Pixelwerte umfasst, kann beispielsweise einer Informationslücke in der Sinogramminformation entsprechen, die fehlender Information aus einer Zeile oder einer Spalte entspricht. Eine Lücke, die mehrere Pixel umfasst, kann einem "Patch" bzw. einem Pixelbereich entsprechen. Ein Patch ist beispielsweise ein Gebiet an Pixelwerten, das beispielsweise eine Ausdehnung in zwei Koordinatenrichtungen des Sinogramms umfasst. Wie oben erwähnt, kann ein zweidimensionales Sinogramm oder ein erweitertes 3D-Sinogramm gemeint sein. Ein Patch kann beispielsweise eine symmetrische Ausdehnung um die Ortskoordinate der Lücke haben, oder eine asymmetrische Ausdehnung um die Ortskoordinate der Lücke. Eine Ausdehnung kann beispielsweise in einer Koordinatenrichtung des Sinogramms (zum Beispiel einer Position entlang eines linearen Bildsensors) größer sein als in der anderen Richtung des Sinogramms (beispielsweise einer Winkelrichtung). Eine Ausdehnung kann beispielsweise in beiden Koordinatenrichtungen des Sinogramms gleich groß sein. Eine Ausdehnung bezieht sich beispielsweise auf die Anzahl der Pixel, bzw. Pixelwerte in einer Koordinatenrichtung. Koordinaten beziehen sich beispielsweise auf einen Winkelschritt der Winkelprojektionen eines Dual Energy CTs. Daher kann die Verarbeitungsvorrichtung die lückenbehaftete zweite Sinogramminformation verarbeiten und unter Verwendung der ersten Sinogramminformation und der zweiten Sinogramminformation die Information der Lücke synthetisieren. Eine Lücke kann beispielsweise mehr als ein Pixel (Pixelwert / Grauwert) umfassen. Eine Lücke kann beispielsweise 99% oder weniger der zweiten Sinogramminformation umfassen im Vergleich zur ersten Sinogramminformation. Eine Lücke kann beispielsweise 95% oder weniger der zweiten Sinogramminformation umfassen im Vergleich zur ersten Sinogramminformation. Eine Lücke kann beispielsweise 90%, 85%, 75%, 70%, 60%, 50%, 40%, 30%, 20%, 10% oder weniger der zweiten Sinogramminformation umfassen im Vergleich zur ersten Sinogramminformation. Eine Lücke kann beispielsweise 1% oder mehr der zweiten Sinogramminformation umfassen im Vergleich zur ersten Sinogramminformation. Eine Lücke kann beispielsweise 50% oder mehr der zweiten Sinogramminformation umfassen im Vergleich zur ersten Sinogramminformation. Das bedeutet, dass beispielsweise 90% der Winkelprojektionen, die in der ersten Sinogramminformation vorhanden sind, in der zweiten Sinogramminformation nicht umfasst sind. Eine Lücke entspricht dann beispielsweise 90% der zweiten Sinogramminformation im Vergleich zu beispielsweise Winkelprojektionen der ersten Sinogramminformation. Eine Lücke kann sich (auch) auf das Fehlen von Daten oder Pixel in der zweiten Sinogramminformation im Vergleich zur ersten Sinogramminformation beziehen. Entsprechend der gewünschten oder der benötigten Qualität der Dual-Energy-Methode kann eine Lücke der zweiten Sinogramminformation (beispielsweise eine Größe der Lücke bzw. der Lücken der zweiten Sinogramminformation im Vergleich zu der ersten Sinogramminformation) eingestellt oder pre-determiniert werden. Lücke bezieht sich nicht auf zusammenhängende Information. Eine Lücke bezieht sich beispielsweise auf die zusammen betrachtete fehlende Information einer zweiten Sinogramminformation im Vergleich zu einer ersten Sinogramminformation.

Vorteilhafterweise ist es beispielsweise möglich, mit einer reduzierten Auflösung (zum Beispiel Winkelauflösung) der zweiten Sinogramminformation, die einer Lücke der zweiten Sinogramminformation gegenüber der ersten Sinogramminformation entspricht (bzw. die Lücken in der zweiten Sinogramminformation im Vergleich zu der ersten Sinogramminformation zur Folge hat), eine Dual-Energy-Methode durchzuführen, die einem mit einer ohne auflösungsreduzierten Dual-Energy-Methode erreichten Ergebnis zumindest im Wesentlichen entspricht.

Gemäß einer bevorzugten Ausführungsform kann die Verarbeitungsvorrichtung dazu ausgelegt sein, um die Lückenfüller-Sinogramminformation zu erhalten, um eine auflösungs-erhöhte Version der zweiten Sinogramminformation zu erhalten. Eine auflösungs-erhöhte Version kann beispielsweise eine höhere Winkel-Auflösung (als bei der als Eingangsgröße erhaltenen zweiten Sinogramminformation vorhanden) aufweisen. Beispielsweise kann eine auflösungs-erhöhte Version der zweiten Sinogramminformation erhalten werden, indem die Verarbeitungsvorrichtung dazu ausgelegt ist, eine (Winkel-)Lücke der zweiten Sinogramminformation mit Lückenfüller-Sinogramminformation zu füllen, indem beispielsweise die Lückenfüller-Sinogramminformation der zweiten Sinogramminformation hinzugefügt wird. Daher ist es möglich, eine auflösungs-erhöhte zweite Sinogramminformation unter Verwendung einer erfindungsgemäßen Verarbeitungsvorrichtung gemäß Ausführungsformen zu erhalten, basierend auf erster Sinogramminformation und (auflösungs-reduzierter) zweiter Sinogramminformation. Eine vom Benutzer eines Dual-Energy-CT beabsichtigt reduzierte Auflösung einer zweiten Sinogramminformation kann vorteilhafterweise zu einer geringeren Aufnahmezeit und/oder zu einer geringeren Strahlendosis führen.

Gemäß einer bevorzugten Ausführungsform kann die erste Sinogramminformation eine erste Winkelauflösung aufweisen. Die zweite Sinogramminformation kann eine zweite Winkelauflösung aufweisen, die kleiner ist als die erste Winkelauflösung. Die Verarbeitungsvorrichtung kann ausgelegt sein, um die Lückenfüller-Sinogramminformation für einen Winkelwert zu erhalten, der zwischen zwei Winkelwerten der zweiten Sinogramminformation liegt.

Um eine von der Verarbeitungsvorrichtung erhaltene Sinogramminformation (Input- oder Eingangs-Sinogramminformation) von einer Sinogramminformation zu unterscheiden, die unter Verwendung einer Lückenfüller-Sinogramminformation erhalten wird, kann Sinogramminformation, die Lückenfüller-Sinogramminformation enthält, als Ausgangs-Sinogramminformation bezeichnet werden. Vorteilhafterweise kann daher bei einer beispielsweise um 50% reduzierten Auflösung der zweiten Sinogramminformation eine Aufnahmezeit für die zweite Sinogramminformation bei einer sequentiellen Aufnahme um 50% reduziert werden.

Gemäß einer bevorzugten Ausführungsform kann die zweite Sinogramminformation zum Erhalten der Lückenfüller-Sinogramminformation (zweite) Sinogramminformation sein, die Sinogramminformationsnachbarn der zweiten Sinogramminformation zur Lücke umfasst.

Je nach Lücke wird von Sinogramminformationsnachbarn ein entsprechendes Set an Pixeln umfasst. Beispielsweise können Sinogramminformationsnachbarn für eine Lücke, die ein Pixel umfasst, die Pixel umfassen, die sich in direkter Nachbarschaft zur Lücke befinden. Dabei kann eine Nachbarschaft mit einem beispielsweise Pixel-weisen, absoluten oder relativen Abstand von der Lücke definiert sein. Sinogramminformationsnachbarn können daher beispielsweise Pixel sein, die direkt an ein Pixel einer Lücke anschließen und/oder Pixel sein, die mit mindesten einem Pixel oder mehreren Pixeln dazwischen von der Lücke beabstandet sind. Eine Sinogramminformationsnachbarschaft kann beispielsweise Pixel mit einem maximalen Pixel-weisen bzw. in Pixelabständen definierten Abstand von der Lücke umfassen, wobei ein maximaler Pixel-weiser Abstand ein Abstand mit einer ganzzahligen Anzahl an Pixeln in beispielsweise einer oder mehreren Richtungen im Pixelkoordinatensystem sein kann. Eine Sinogramminformationsnachbarschaft kann in einer ersten Koordinatenrichtung einen anderen oder einen gleichen Pixel-weisen Abstand umfassen als/wie in einer anderen, zweiten Koordinatenrichtung. Entsprechendes ist auf Lücken anwendbar, die einer Pixellinie oder einem Pixelpatch entsprechen. Vorteilhafterweise kann eine Lückenfüller-Sinogramminformation entsprechend den Sinogramminformationsnachbarn rekonstruiert, bzw. synthetisiert werden. Sinogramminformationsnachbarn können je nach Lücke und/oder zu untersuchendem Objekt anders, beispielsweise von einem Benutzer, definiert werden, um eine Qualität einer Lückenfüller-Sinogramminformation zu erhöhen. Sinogramminformationsnachbarn können per Lücke anders gewählt sein.

Vorteilhafterweise kann beispielsweise strukturelle Information, die in einer ersten Sinogramminformation codiert ist, für die Rekonstruktion bzw. Synthese von Lückenfüller-Sinogramminformation, die eine Lücke in der zweiten Sinogramminformation füllt, genutzt werden. Ein Abstand von einer Lücke in einer Richtung kann beispielsweise kein Pixel (kein Pixel nur für den Abstand von einer Lücke bei erster Sinogramminformation), ein Pixel oder mehr als ein Pixel umfassen. Ein Abstand von einer Lücke in einer Richtung kann beispielsweise weniger als 100, weniger als 50, weniger als 20 oder weniger als 10 Pixel umfassen. Sinogramminformationsnachbarn können für eine erste Sinogramminformation weniger oder mehr Pixel umfassen als für eine zweite Sinogramminformation. Vorteilhafterweise kann der Einfluss einer ersten Sinogramminformation und/oder einer zweiten Sinogramminformation beim Erhalten einer Lückenfüller-Sinogramminformation durch die Verarbeitungsvorrichtung mit den von Sinogramminformationsnachbarn umfassten Pixeln der ersten Sinogramminformation und der zweiten Sinogramminformation eingestellt werden. Ein weiterer Vorteil kann sein, dass eine Verschiebung der Pixel der ersten Sinogramminformation zu Pixeln der zweiten Sinogramminformation, die beispielsweise bei sequentieller Aufnahme der jeweiligen Sinogramminformation entstehen kann, durch eine geeignete Wahl oder Pre-Determinierung von Sinogramminformationsnachbarn zumindest teilweise kompensiert werden kann.

Gemäß einer bevorzugten Ausführungsform kann die Verarbeitungsvorrichtung dazu ausgelegt sein, die Lückenfüller-Sinogramminformation basierend auf einem oder mehreren der Lücke benachbarten Sinogrammwerten (Sinogramminformationsnachbarn) der zweiten Sinogramminformation und zusätzlich basierend auf einem oder mehreren der Lücke benachbarten Sinogrammwerten (Sinogramminformationsnachbarn) der ersten Sinogramminformation und/oder einem oder mehreren der Lücke zugeordneten Sinogrammwerten der ersten Sinogramminformation zu erhalten.

Benachbart kann sich dabei beispielsweise auf eine winkelmäßige oder eine örtliche Nachbarschaft beziehen. Ein Winkelschritt in der CT-Bildgebung ist bekanntermaßen beispielsweise kleiner, bzw. deutlich kleiner als 1°. Eine Lücke entsteht beispielsweise, wenn eine zweite Sinogramminformation mit einer reduzierten Abtastung (z.B. einer reduzierten Winkelauflösung) im Vergleich zu einer ersten Sinogramminformation aufgenommen wird. Unter einer reduzierten Abtastung ist beispielsweise eine Abtastung mit größeren Winkelschritten zu verstehen.

Gemäß einer bevorzugten Ausführungsform kann die erste Sinogramminformation zum Erhalten der Lückenfüller-Sinogramminformation erste Sinogramminformation sein, die Sinogramminformationsnachbarn der ersten Sinogramminformation zur Lücke und erste Sinogramminformation der Lücke umfasst.

Beispielsweise kann eine Information, die die Sinogramminformationsnachbarn betrifft, in der jeweiligen Sinogramminformation umfasst sein. Vorteilhafterweise kann eine Verarbeitungsvorrichtung je nach Abtastung der zweiten Sinogramminformation, Informationen zu Sinogramminformationsnachbarn der zweiten Sinogramminformation erhalten. Die Informationen zu den Sinogramminformationsnachbarn der zweiten Sinogramminformation können in den von der Verarbeitungsvorrichtung erhaltenen zweiten Sinogramminformation umfasst sein. Beispielsweise kann die zweite Sinogramminformation pixelweise oder in mehreren Pixeln umfassenden Patches auf Lücken untersucht werden und/oder die vom Scan bekannten Lücken herangezogen werden. Für eine entsprechende Lücke können dann beispielsweise Sinogramminformationsnachbarn bestimmt werden, die für ein Ermitteln der Lückenfüller-Sinogramminformation vorteilhaft sind. Die Verarbeitungsvorrichtung kann entsprechend dafür konfiguriert sein.

Gemäß einer bevorzugten Ausführungsform kann die Verarbeitungsvorrichtung dazu ausgelegt sein, die erste Sinogramminformation und die zweite Sinogramminformation gleichzeitig mit einem Scan oder sequenziell mit zwei Scans zu erhalten.

Wie oben bereits beschrieben, sind unterschiedliche Systeme bekannt. Ein Dual-Energy-CT kann entsprechende Hardware aufweisen, mit der eine erste Sinogramminformation und eine zweite Sinogramminformation gleichzeitig erfasst werden können oder kann Hardware aufweisen, die nur einen Scan bei einem spektralen Parameter durchführen kann. Eine erste Sinogramminformation und eine zweite Sinogramminformation kann daher zeitgleich oder zeitversetzt erhalten werden. Vorteilhafterweise kann die Verarbeitungsvorrichtung konfiguriert sein, um in den Fällen jeweils eine Lückenfüller-Sinogramminformation zu erhalten (zu ermitteln). Für gleichzeitig erhaltene Sinogramminformation kann die Verarbeitungsvorrichtung durch Bestimmung einer lückenbehafteten zweiten Sinogramminformation vorteilhafterweise eine Strahlendosis verringern, wobei die Lückenfüller-Sinogramminformation von der Verarbeitungsvorrichtung (zum Beispiel auf rechnerischem Wege) erhalten wird (ermittelt wird). Ein weiterer Scan zur Korrektur und/oder Vervollständigung der lückenbehafteten zweiten Sinogramminformation ist nicht nötig, bzw. entfällt. Für sequentiell erhaltene Sinogramminformation kann die Verarbeitungsvorrichtung durch Bestimmung einer lückenbehafteten zweiten Sinogramminformation (Beispielsweise durch Bestimmung der zweiten Sinogramminformation mit verringerter Anzahl an Winkelschritten) vorteilhafterweise eine Strahlendosis und/oder eine Aufnahmezeit verringern. Ein weiterer Scan zur Korrektur und/oder Vervollständigung der lückenbehafteten zweiten Sinogramminformation ist nicht nötig, bzw. entfällt. Die erhaltenen (ermittelte oder synthetisierte) Sinogramminformation kann vorteilhafterweise eine von einem Scan erhaltene Sinogramminformation ersetzen und/oder ergänzen.

Gemäß einer bevorzugten Ausführungsform kann die erste Sinogramminformation eine Mehrzahl von Durchstrahlungsbild-Zeilen oder Durchstrahlungsbildern umfassen, wobei die Durchstrahlungsbilder verschiedenen (Durchstrahlungs-)Winkeln zugeordnet sind und beispielsweise einer ersten Durchleuchtungs-Spitzen-Wellenlänge oder einer ersten Durchleuchtungs-Beschleunigungsspannung zugeordnet sind. Die zweite Sinogramminformation kann eine Mehrzahl von Durchstrahlungsbild-Zeilen oder Durchstrahlungsbildern umfassen, die verschiedenen Durchstrahlungswinkeln zugeordnet sind und beispielsweise einer zweiten Durchleuchtungs-Spitzen-Wellenlänge oder einer zweiten Durchleuchtungs-Beschleunigungsspannung zugeordnet sind, wobei die erste Sinogramminformation und die zweite Sinogramminformation unterschiedlichen Durchstrahlungs-Strahlenergien einer Dual-Energy-CT (Mehr-Energie-Computertomographie) zugeordnet sind.

Unterschiedliche Durchstrahlungsenergien können beispielsweise durch Verwendung unterschiedlicher Beschleunigungsspannungen und/oder unter Verwendung verschiedener Vorfilter oder zusätzlicher Vorfilter erreicht werden. Dual-Energy-CT kann eine Bezeichnung für einen Mehr-Energien-Computertomographen oder für Mehr-Energien-Computertomographie sein. Mehr-Energien bezieht sich darauf, dass der Computertomograph dazu ausgelegt ist, unter Verwendung von mehr als einem spektralen Parameter eine Computertomographie durchzuführen.

Die Verarbeitungsvorrichtung kann zum Erhalten der Lückenfüller-Sinogramminformation ein mathematisches Verfahren, wie beispielsweise einen polynomialen Fit oder ein neuronales Netz verwenden. Die Verarbeitungsvorrichtung kann konfiguriert sein, zum Erhalten einer Lückenfüller-Sinogramminformation ein oder mehrere mathematische Verfahren und/oder ein neuronales Netz zu verwenden.

Bei einem mathematischen Verfahren, wie beispielsweise einem polynomialen Fit, können beispielsweise fünf Projektionen als Eingangsdaten verwendet werden. Im Folgenden wird ein Beispiel mit HE Projektionen (z.B. Projektionen mit hoher Energie bzw. hoher Beschleunigungsspannung) als zweiter Sinogramminformation und LE Projektionen (z.B. Projektionen mit vergleichsweise niedrigerer Energie bzw. vergleichsweise niedrigerer Beschleunigungsspannung) als erster Sinogramminformation ausgeführt. Dies ist lediglich zur Veranschaulichung gedacht und es ist selbstverständlich, dass HE Projektionen auch als erste Sinogramminformation und LE Projektionen als zweite Sinogramminformation vorliegen können.

Gemäß einer bevorzugten Ausführungsform kann die Verarbeitungsvorrichtung dazu ausgelegt sein, die Lückenfüller-Sinogramminformation unter Verwendung eines neuronalen Netzes zu erhalten.

Beispielsweise kann ein DNN (deep neural network bzw. "tiefes neuronales Netz"), wie beispielsweise ein GAN (Generative Adversarial Network bzw. "erzeugendes gegnerisches Netzwerk"), ein CNN (convolutional neural network bzw. "faltendes neuronales Netzwerk"), eine U-Net Architektur ("U-Netz-Architektur") oder eine Kombination aus einem oder mehreren der genannten verwendet werden, um Lückenfüller-Sinogramminformation zu erhalten. Diese können beispielsweise dazu ausgelegt sein, um Pixel für Pixel, Patch für Patch oder Bild für Bild die Lückenfüller-Sinogramminformation zu erhalten. Die Lückenfüller-Sinogramminformation kann beispielsweise erhalten werden, indem die erste und die zweite Sinogramminformation (oder ein Ausschnitt daraus) als Eingangsinformation dem neuronalen Netz zur Verfügung gestellt wird. Das neuronale Netz kann beispielsweise aus den strukturellen Informationen der ersten Sinogramminformation strukturelle Information der zweiten Sinogramminformation erzeugen, so dass eine Lückenfüller-Sinogramminformation erhalten wird. Das neuronale Netz kann dabei ausgelegt sein, die zweite Sinogramminformation derart mit Lückenfüller-Sinogramminformation zu ergänzen, dass eine Auflösung der zweiten Sinogramminformation einer Auflösung der ersten Sinogramminformation zumindest im Wesentlichen entspricht. Aus der gewonnenen zweiten Sinogramminformation (Ausgangs-Sinogramminformation) und der ersten Sinogramminformation kann vorteilhafterweise beispielsweise Materialinformation über das untersuchte Objekt erlangt werden und/oder es können beispielsweise Strahlaufhärtungsartefakte reduziert werden. Die Verarbeitungsvorrichtung kann vorteilhafterweise dazu ausgelegt sein, aus Ausgangs-Sinogramminformation und erster Sinogramminformation Materialinformation über das untersuchte Objekt zu erlangen und/oder Strahlhärtungsartefakte zu reduzieren.

Vorteilhafterweise kann durch den Einsatz eines neuronalen Netzes die zweite Sinogramminformation erhalten (erzeugt oder synthetisiert) werden, die einer zweiten Sinogramminformation, die ohne Lücken erzeugt wurde, zumindest im Wesentlichen entspricht. Durch bewusste Bestimmung der zweiten Sinogramminformation mit Lücken (z.B. mit verringerter Winkelauflösung) kann vorteilhafterweise eine Scanzeit reduziert werden und/oder eine Strahlendosis reduziert werden, und die Lücken können dann, beispielsweise unter Verwendung des neuronalen Netzwerks, rechnerisch aufgefüllt werden. Im Übrigen wurde erkannt, dass ein neuronales Netzwerk besonders gut geeignet ist, um Abhängigkeiten zwischen der ersten Sinogramminformation und der zweiten Sinogramminformation bei der Füllung der Lücken auszunutzen. So kann das neuronale Netzwerk beispielsweise mit lückenfreien Sinogramminformationen trainiert (und beispielsweise an die Eigenschaften des zu analysierenden Objekts bzw. einer entsprechenden Objektklasse, z.B. Mensch oder technischer Gegenstand, angepasst werden, oder auch an die spezifischen Gegebenheiten einer CT-Anordnung angepasst) werden. Somit kann das neuronale Netzwerk nach Abschluss des Trainings die Lücken in der zweiten Sinogramminformation besonders zuverlässig und mit hoher Genauigkeit auffüllen, wodurch ein Verlust an Auflösung bzw. an Schärfe durch die Lücken in der ursprünglichen zweiten Sinogramminformation niedrig gehalten werden kann.

Gemäß Ausführungsformen kann das neuronale Netz mindestens eines aus CNN, GAN und U-Net umfassen.

Beispielsweise kann mindestens eines der genannten neuronalen Netze oder Architekturen verwendet werden, oder es können sogar mindestens zwei der genannten neuronalen Netze oder Architekturen hintereinander geschaltet oder parallel verschaltet sein. Es können verschiedene oder gleichartige neuronale Netze miteinander verschaltet sein und/oder aufeinander folgen. Ausführungsformen der Erfindung sind nicht auf die genannten neuronalen Netz Architekturen beschränkt. Es können andere Architekturen für ein neuronales Netz eingesetzt werden. Insbesondere hat sich gezeigt, dass die genannten Typen von neuronalen Netzen besonders gut für die vorliegende Anwendung geeignet sind.

Auf diese Weise können Pixel für Pixel, Patch für Patch oder Bild für Bild die Projektionen einer zweiten Sinogramminformation generiert werden bzw. die Lücken im Sinogramm der zweiten Sinogramminformationen geschlossen werden, wobei die strukturelle Information über das Objekt bereits aus dem Scan der ersten Sinogramminformation bekannt ist und nur die energiespezifischen Unterschiede generiert werden müssen.

Gemäß Ausführungsformen kann zunächst die erste Sinogramminformation für alle Winkelschritte gemessen werden. Im nächsten Schritt kann die zweite Sinogramminformation unter Auslassen beispielsweise jeden zweiten Winkelschrittes (also mit halber Winkelauflösung) aufgenommen werden. In anderen Worten kann die zweite Sinogramminformation für beispielsweise jeden zweiten Winkelschritt, dritten Winkelschritt, vierten Winkelschritt, fünften Winkelschritt oder n-ten Winkelschritt gemessen werden Es sind auch Kombinationen verschiedener Winkelschritte möglich. Um beispielsweise das Trainieren des neuronalen Netzwerkszuzulassen, können beispielsweise einige Projektionen für die fehlenden Winkelschritte aufgenommen werden. Dann liegen genug Daten vor um einen Trainingsdatensatz bereitzustellen.

Zum Trainieren des Neuronalen Netzwerks können die Trainings-Daten beispielsweise augmentiert und/oder weitere Techniken zur Verhinderung von Overfitting (Überbestimmung) angewendet werden, wie beispielsweise Regularization (Regularisierung), Dropout (Weglassen von Werten), Noise (Rauschen), Early Stopping (Frühes Anhalten).

Die beim Trainieren des Neuronalen Netzwerks optimierten Parameter, können beispielsweise entweder zufällig oder durch Parameter aus einem vor-trainierten Netzwerk mit der gleichen Architektur, welches erfolgreich zur Lösung eines ähnlichen Problems trainiert wurde, initialisiert werden (Transfer learning bzw. Ubertragungs-Lernen).

Weitere Details bezüglich einiger hier erwähnter Techniken aus den Bereichen Machine Learning (Maschinenlernen) / Deep Learning (Tiefes Lernen) sind in der Fachliteratur zu finden und können hier ebenfalls angewendet werden.

Die hier beispielhaft beschriebene Architektur des CNN ist eine primitive Realisation des Lösungswegs. In der praktischen Umsetzung hat sich ein auf der U-Net-Architektur basierendes neuronales Netzwerk als akkurat erwiesen. Allerdings ist es ebenfalls möglich, andere Netzwerkarchitekturen einen neuronalen Netzwerks zu verwenden, die in manchen Fällen die Effizienz des hier beschriebenen U-Nets übertreffen können. Diese können beispielsweise adaptiert werden, um erfindungsgemäß Lückenfüller-Sinogramminformation zu erhalten.

Vorteilhafterweise ist das neuronale Netz derart trainiert und/oder organisiert, dass es unter Verwendung von erster und zweiter Sinogramminformation eine Lückenfüller-Sinogramminformation erhalten kann.

Eine weitere Ausführungsform schafft einen Computertomographen umfassend mindestens eine Verarbeitungsvorrichtung, beispielsweise gemäß den hierin offenbarten Ausführungsformen.

Vorteilhafterweise kann der Computertomograph unter Verwendung der Verarbeitungsvorrichtung eine lückenbehaftete zweite Sinogramminformation vervollständigen (unter Verwendung der ersten Sinogramminformation und der zweiten Sinogramminformation). Der Computertomograph kann vorteilhafterweise, je nach Ausführung des Computertomographen mit einem Strahler und einem Detektor oder mit mehreren Strahlern und entsprechend mehreren Detektoren, zumindest im Wesentlichen eine Strahlendosis für beispielsweise einen Patienten oder ein Objekt verringern und/oder eine Aufnahmezeit von Dual-Energy-CTs zumindest im Wesentlichen verringern.

Eine weitere Ausführungsform stellt ein Verfahren zum Erhalten von Lückenfüller-Sinogramminformation bereit. Das Verfahren umfasst das Erhalten einer ersten Sinogramminformation, wobei die erste Sinogramminformation einem ersten spektralen Parameter zugeordnet ist, das Erhalten einer zweiten Sinogramminformation, wobei die zweite Sinogramminformation einem zweiten spektralen Parameter zugeordnet ist (wobei sich der zweite spektrale Parameter von dem ersten spektralen Parameter unterscheidet). Das Verfahren umfasst ferner das Ermitteln einer Lückenfüller-Sinogramminformation, die eine Lücke in der zweiten Sinogramminformation füllt, basierend auf der ersten Sinogramminformation und der zweiten Sinogramminformation.

Diese Ausführungsform basiert auf der Erkenntnis, dass eine strukturelle Information des erfassten Objekts in der ersten Sinogramminformation enthalten sein kann und eine Lücke in einer zweiten Sinogramminformation basierend auf der ersten Sinogramminformation und struktureller Information, die in den Teilen der zweiten Sinogramminformation, enthalten sein kann, die keine Lücken aufweist, ermittelt werden kann. Dies kann beispielsweise durch mathematische Methoden oder durch den Einsatz eines neuronalen Netzes erreicht werden.

Gemäß einer bevorzugten Ausführungsform enthält die zweite Sinogramminformation weniger Information als die erste Sinogramminformation. Weniger Information kann beispielsweise bedeuten, dass bei einem Aufnahmeverfahren zum Erhalten einer ersten Sinogramminformation und einer zweiten Sinogramminformation ein Winkelschritt bei der Aufnahme der zweiten Sinogramminformation größer ist, als ein Winkelschritt bei der Aufnahme einer ersten Sinogramminformation. Bei einem Dual-Energy-CT, das erste Sinogramminformation und zweite Sinogramminformation gleichzeitig erzeugen kann, kann weniger Information bedeuten, dass die zweite Sinogramminformation mit weniger Detektorpixeln als die erste Sinogramminformation erzeugt wird. Beispielsweise können bei der zweiten Sinogramminformation (mehr) defekte Pixel vorhanden sein. Vorteilhafterweise können defekte Pixel ("bad pixel") durch das Erhalten einer Lückenfüller-Sinogramminformation kompensiert werden.

Gemäß einer bevorzugten Ausführungsform kann das Ermitteln der Lückenfüller-Sinogramminformation erste Sinogramminformation und zweite Sinogramminformation umfassen. Dabei kann die erste Sinogramminformation und die zweite Sinogramminformation jeweils räumliche Sinogramminformationsnachbarn zur Lücke der zweiten Sinogramminformation umfassen (bzw. verwenden) und die erste Sinogramminformation kann (auch / zusätzlich) erste Sinogramminformation der Lücke (die der Lücke der zweiten Sinogramminformation entspricht) umfassen. Das bedeutet, dass der Datensatz der ersten Sinogramminformation an der räumlichen Stelle der Lücke der zweiten Sinogramminformation Information aufweist, die erste Sinogramminformation ist und dem ersten spektralen Parameter zugeordnet ist. Die Information der Lücke, die erste Sinogramminformation ist (die nur bei der ersten Sinogramminformation vorhanden ist) kann verwendet werden, um Lückenfüller-Sinogramminformation (zweite Sinogramminformation der Lücke) zu erhalten. Vorteilhafterweise kann beispielsweise ein Einfluss der ersten Sinogramminformation und der zweiten Sinogramminformation über die Sinogramminformationsnachbarn der jeweiligen Sinogramminformation gesteuert werden.

Gemäß einer bevorzugten Ausführungsform kann die erste Sinogramminformation eine erste Winkelauflösung aufweisen und die zweite Sinogramminformation kann eine zweite Winkelauflösung aufweisen, die kleiner ist als die erste Winkelauflösung. Das Ermitteln der Lückenfüller-Sinogramminformation kann ferner ein Füllen der Lücke (in der zweiten Sinogramminformation) mit Lückenfüller-Sinogramminformation umfassen. Dabei kann beispielsweise eine aus erster und zweiter Sinogramminformation erhaltene Lückenfüller-Sinogramminformation an der (räumlichen) Stelle der Lücke (der zweiten Sinogramminformation) an der (räumlichen) Stelle in die zweite Sinogramminformation eingefügt werden. Beispielsweise kann dadurch eine Auflösung der zweiten Sinogramminformation an der Stelle der Lücke erhöht werden. Beispielsweise kann dadurch, trotz Lücke(n) in der zweiten Sinogramminformation, der Vorteil eines Dual-Energy-CT zumindest im Wesentlichen erhalten bleiben.

Gemäß einer bevorzugten Ausführungsform können die räumlichen Sinogramminformationsnachbarn Sinogramminformation in einer Umgebung $(2p + 1) \times (2q + 1)$ umfassen. Und Sinogramminformationsnachbarn der ersten Sinogramminformation Sinogramminformation (mit Koordinaten) $([x +/- p, y +/- q, a])$, und Sinogramminformationsnachbarn der zweiten Sinogramminformation Sinogramminformation (mit Koordinaten) $([x +/- p, y +/- q, \alpha - r])$ und $([x +/- p, y +/- q, \alpha + r])$ umfassen. Dabei kann p eine pixelweise Abweichung in eine erste Richtung x, die im Wesentlichen senkrecht zu einem Projektionswinkel $\alpha$ ist, q eine pixelweise Abweichung in eine zweite Richtung y, die im Wesentlichen parallel zu einer Rotationsachse des Projektionswinkels $\alpha$ und zur ersten Richtung x und r eine Winkelabweichung, beispielsweise in Grad oder Winkelschritten, sein. Folglich bezeichnen x +/- p und y +/- q hier einen rechteckigen Bildausschnitt, und zwar in x-Richtung von der Position x-p bis x+p, in y-Richtung von y-q bis y+q. Es können auch andere Koordinatenkonventionen verwendet werden. Dabei kann das Ermitteln der Lückenfüller-Sinogramminformation auf Sinogramminformationsnachbarn der ersten und der zweiten Sinogramminformation basieren. Beispielsweise können p, q und r natürliche Zahlen sein, für den Fall, dass r einen Winkelschritt bezeichnet, andernfalls kann r ein Winkel in Grad sein. r kann beispielsweise vom Winkelauflösungsvermögen des Dual-Energy-CT abhängen. Ein Einzelpixel kann beispielsweise mit $([x, y, a])$, eine Linie beispielsweise mit $([x, y +/- q, a])$ oder $([x +/- p, y, a])$ bezeichnet (identifiziert) werden, Patches entsprechend mit $([x +/- p, y +/- q, \alpha])$. p und q können gleich oder unterschiedlich sein. Durch ein gleiches p und q können beispielsweise symmetrische Sinogramminformationsnachbarn, durch unterschiedliche Werte für p und q können asymmetrische Sinogramminformationsnachbarn definiert sein. Vorteilhafterweise können durch ein Wählen der Werte die Eingangsdaten angepasst werden. Es ist beispielsweise möglich, nicht nur ein einziges fehlendes Pixel zur gleichen Zeit zu erzeugen, sondern eine zusammenhängende Fläche von Pixeln (beispielsweise Linien oder Patches), beispielsweise rechteckige Bildausschnitte. Das ändert wenig an der formalen Schreibweise wie oben verwendet, beispielsweise sind x und y dann keine Koordinaten eines Einzelpixels, sondern zusammenhängende Intervalle. Beispielsweise kann die Verarbeitungsvorrichtung gemäß Ausführungsformen dazu ausgelegt sein, Werte für p, q und r zu wählen, bevorzugter Weise für eine und/oder mehrere Lücken. Beispielsweise können (unregelmäßige) Lücken mit einer konstanten oder einer variablen Anzahl an Sinogramminformationsnachbarn in die jeweiligen Richtungen umgeben sein. Beispielsweise kann so eine L-förmige Lücke mit beispielsweise Sinogramminformationsnachbarn umfassend jeweils 2 Pixel in jede Richtung von der Lücke weg definiert sein. Andere (unregelmäßige) Lückenformen können vorkommen. Dies kann den Vorteil haben, dass Sinogramminformationsnachbarn an die Lücke angepasst sind und eine Qualität der Lückenfüller-Sinogramminformation beim Ermitteln oder Erhalten erhöht wird.

Gemäß einer bevorzugten Ausführungsform kann das Verfahren ferner das Entfernen von Strahlaufhärtungsartefakten basierend auf der ersten Sinogramminformation, der zweiten Sinogramminformation und der Lückenfüller-Sinogramminformation, und/oder das Ermitteln von Materialinformationen des untersuchten Objekts, basierend auf der ersten Sinogramminformation, der zweiten Sinogramminformation und der Lückenfüller-Sinogramminformation, umfassen. Das Entfernen von Strahlaufhärtungsartefakten kann beispielsweise von einem Strahlaufhärtungs-Entfernungsmodul vorgenommen werden. Beispielsweise kann das Strahlaufhärtungs-Entfernungsmodul dazu eingerichtet sein, basierend auf der ersten Sinogramminformation, der zweiten Sinogramminformation und der Lückenfüller-Sinogramminformation, einen Stahlaufhärtungseffekt zu reduzieren. Das Strahlaufhärtungs-Entfernungsmodul kann beispielsweise ein integraler Teil der Verarbeitungsvorrichtung sein. Alternativ kann das Strahlaufhärtungs-Entfernungsmodul ein separates Modul sein, das in Datenkommunikation mit der Verarbeitungsvorrichtung steht. Das Ermitteln von Materialinformationen kann beispielsweise von einem Materialinformations-Ermittlungsmodul vorgenommen werden. Das Materialinformations-Ermittlungsmodul kann beispielsweise dazu ausgelegt sein, basierend auf der ersten Sinogramminformation, der zweiten Sinogramminformation und der Lückenfüller-Sinogramminformation, eine oder mehrere Materialinformationen zu dem untersuchten Objekt zu ermitteln. Das Materialinformations-Ermittlungsmodul kann beispielsweise ein integraler Teil der Verarbeitungsvorrichtung sein. Alternativ kann das Materialinformations-Ermittlungsmodul ein separates Modul sein, das in Datenkommunikation mit der Verarbeitungsvorrichtung steht.

Vorteilhafterweise kann die Qualität eines Entfernens von Strahlaufhärtungsartefakten und/oder das Ermitteln von Materialinformationen im Wesentlichen bei lückenbehafteter zweiter Sinogramminformation gleich hoch sein, wie bei lückenunbehafteter zweiter Sinogramminformation. Dadurch kann beispielsweise eine Aufnahmezeit und/oder eine Strahlendosis beim Ermitteln von Materialinformationen und/oder beim Entfernen von Strahlaufhärtungsartefakten verringert werden.

Gemäß einer bevorzugten Ausführungsform kann das Verfahren vor dem Ermitteln der Lückenfüller-Sinogramminformation ein Ermitteln einer Registrierung der ersten Sinogramminformation auf die zweite Sinogramminformation und das Durchführen einer Registrierung der ersten Sinogramminformation auf die zweite Sinogramminformation umfassen. Dies kann beispielsweise der Fall sein, wenn eine erste Sinogramminformation und eine zweite Sinogramminformation sequentiell erhalten werden. Ein Objekt, das beispielsweise ein Lebewesen sein kann, kann sich in der Zeit zwischen dem Erhalten der ersten Sinogramminformation und dem Erhalten der zweiten Sinogramminformation beispielsweise bewegt haben oder eine Atembewegung ausgeführt haben, die zu einer relativen Verschiebung der erhaltenen Information führen kann. Mit Mitteln der Registrierung kann dieser Versatz in den Informationen zumindest im Wesentlichen ausgeglichen werden.

Eine weitere Ausführungsform der Erfindung stellt ein Computerprogrammprodukt bereit. Das Computerprogrammprodukt umfasst Befehle, die bei der Ausführung des Programms durch einen Computer diesen veranlassen, das Verfahren gemäß Ausführungsformen der Erfindung auszuführen.

Gemäß Ausführungsformen der Erfindung wird die Aufgabe durch ein Computerlesbares (Speicher)medium gelöst. Das Computerlesbare (Speicher)medium umfasst Befehle, die bei der Ausführung der Befehle durch einen Computer diesen veranlassen, das Verfahren gemäß Ausführungsformen der Erfindung auszuführen.

Eine weitere Ausführungsform des erfindungsgemäßen Verfahrens ist daher ein Datenstrom oder eine Folge von Signalen, die das Computerprogramm zur Durchführung eines der hierin beschriebenen Verfahren darstellen. Der Datenstrom oder die Signalfolge kann beispielsweise so konfiguriert sein, dass er/sie über eine Datenkommunikationsverbindung, z. B. über das Internet, übertragen wird.

Eine weitere Ausführungsform umfasst ein Verarbeitungsmittel, z. B. einen Computer oder ein programmierbares Logikgerät, das so konfiguriert oder angepasst ist, dass es eines der hierin beschriebenen Verfahren durchführt.

Eine weitere Ausführungsform umfasst einen Computer, auf dem das Computerprogramm zur Durchführung eines der hierin beschriebenen Verfahren installiert ist.

Eine weitere Ausführungsform gemäß der Erfindung umfasst eine Vorrichtung oder ein System, die bzw. das konfiguriert ist, um ein Computerprogramm zum Ausführen eines der hierin beschriebenen Verfahren an einen Empfänger zu übertragen (z. B. elektronisch oder optisch). Der Empfänger kann z.B. ein Computer, ein mobiles Gerät, ein Speichergerät oder dergleichen sein. Die Vorrichtung oder das System kann z. B. einen Dateiserver zur Übertragung des Computerprogramms an den Empfänger umfassen.

In einigen Ausführungsformen kann eine programmierbare Logikvorrichtung (z.B. ein feldprogrammierbares Gate-Array (FPGA)) verwendet werden, um einige oder alle Funktionalitäten der hier beschriebenen Verfahren auszuführen.

In einigen Ausführungsformen kann ein feldprogrammierbares Gate-Array mit einem Mikroprozessor zusammenarbeiten, um eines der hier beschriebenen Verfahren durchzuführen.

Im Allgemeinen werden die Methoden vorzugsweise von einem beliebigen Hardware-Gerät ausgeführt. Die oben beschriebenen Ausführungsformen sind lediglich illustrativ für die Prinzipien der vorliegenden Erfindung.

Gemäß einem Aspekt der Erfindung wird die Lückenfüller-Sinogramminformation nicht auf Basis von weiterverarbeiteten Daten, wie beispielsweise Voxeln ermittelt oder berechnet, sondern beispielsweise (unmittelbar) auf der Basis der Sinogramminformation.

**Figurenkurzbeschreibung**

Ausführungsbeispiele gemäß der vorliegenden Erfindung, zum Beispiel eine erfindungsgemäße Verarbeitungsvorrichtung sowie ein erfindungsgemäßer Computertomograph, werden nachfolgend anhand von Zeichnungen näher erläutert. Es zeigen:

Figur 1    Eine schematische Darstellung eines Computertomographen mit einem zu untersuchenden Objekt und einer Verarbeitungsvorrichtung zum Erhalten einer Lückenfüller-Sinogramminformation,

Figur 2    Eine schematische Darstellung eines Computertomographen mit einem zu untersuchenden Objekt und einer Verarbeitungsvorrichtung zum Erhalten einer Lückenfüller-Sinogramminformation,

Figur 3    Eine schematische Darstellung von Winkelschritten bei Projektionen von erster Sinogramminformation und zweiter Sinogramminformation in einer Verarbeitungsvorrichtung mit Erzeugung von Lückenfüller-Sinogramminformation mit einem neuronalen Netz,

Figur 4    Eine Flussdiagramm eines Verfahrens zum Erhalten einer Lückenfüller-Sinogramminformation in einem Blockdiagramm,

Figur 5    Eine schematische Darstellung eines Verfahrens zum Erhalten einer Lückenfüller-Sinogramminformation für eine beispielhafte Lücke, die ein Pixel umfasst.

Figur 6    Eine schematische Darstellung eines Verfahrens zum Erhalten einer Lückenfüller-Sinogramminformation für eine beispielhafte Lücke, die ein Pixellinie umfasst.

Figur 7    Eine schematische Darstellung eines Verfahrens zum Erhalten einer Lückenfüller-Sinogramminformation für eine beispielhafte Lücke, die einen Patch umfasst.

Figur 8    Eine Darstellung von erster Sinogramminformation und zweiter Sinogramminformation zur Verwendung mit einem CNN, beispielhaft mit einem 4x4 Patch für den Output mit zugehörigem 8x8 Patch der Input-Daten eines beispielhaften Winkelschritts.

Figur 9    Ein beispielhafter Fit einer Verteilung mit einem Polynom dritten Grades zum Erhalten einer Abbildungsvorschrift von erster Sinogramminformation zu zweiter Sinogramminformation.

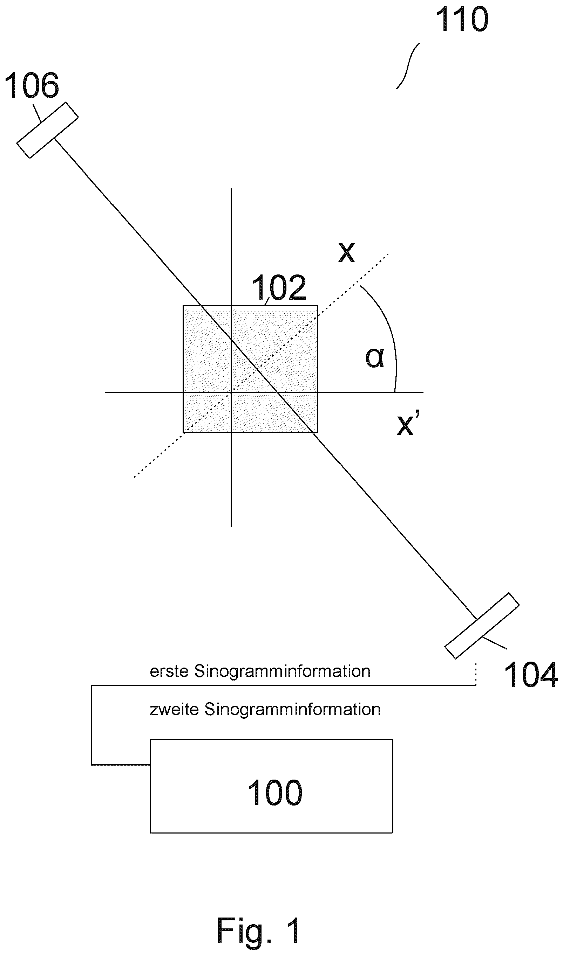

Elemente mit gleicher Funktion und Wirkungsweise sind in den Figuren 1 bis 7 jeweils mit denselben Bezugszeichen versehen.

**Detaillierte Beschreibung der Ausführungsbeispiele**

Nachfolgend werden typische Ausführungsformen der Erfindung anhand der Figuren beschrieben. Bei der Beschreibung typischer Ausführungsformen werden unter Umständen in verschiedenen Figuren und für verschiedene Ausführungsformen gleiche Bezugszeichen für gleiche oder ähnliche Teile verwendet, um die Beschreibung übersichtlicher zu gestalten. Dies bedeutet jedoch nicht, dass entsprechende Teile der Erfindung auf die in den Ausführungsformen dargestellten Varianten beschränkt sind.

## 1. Verarbeitungsvorrichtung zum Erhalten einer Lückenfüller-Sinogramminformation gemäß Figur 1

In der Fig. 1 ist eine beispielhafte Ausführungsform eines Computertomographen mit einer erfindungsgemäßen Verarbeitungsvorrichtung zum Erhalten einer Lückenfüller-Sinogramminformation schematisch gezeigt. Der Computertomograph gemäß Figur 1 ist in seiner Gesamtheit mit 110 bezeichnet. Die Verarbeitungsvorrichtung gemäß Figur 1 ist mit 100 bezeichnet.

Die Verarbeitungsvorrichtung 100 zum Erhalten einer Lückenfüller-Sinogramminformation kann ein integraler Teil des Computertomographen 110 sein oder kann, wie hier gezeigt in Datenkommunikation mit dem Computertomographen 110 sein, um eine erste Sinogramminformation und eine zweite Sinogramminformation zu erhalten. Sinogramminformation wird durch Röntgen-Computertomographie erzeugt. Hier sind, wie oben bereits erwähnt, Verfahren bekannt, wobei eines Sinogramminformation sequentiell erzeugt und ein anderes Sinogramminformation quasi gleichzeitig erzeugt. Figur 1 bezieht sich auf ein Verfahren zum sequentiellen Erzeugen von Sinogramminformation. Dafür sind ein Strahler (Quelle) 106 und ein Detektor 104 schematisch gezeigt, die eine Projektion durch ein zu untersuchendes Objekt 102 ausführen. Das in der Datenaufnahme verwendete Koordinatensystem ist in der Regel an den Röntgenstrahl gekoppelt. Die Ortskoordinate y ist senkrecht zur Zeichenebene (nicht gezeigt). Die Koordinate x rotiert mit $\alpha$ mit ist also fest bezüglich der Röntgenkomponenten 104 und 106, x' ist fest bezüglich des Untersuchungsobjekts 102. Ein Koordinatensystem mit x, y und $\alpha$ referenziert die erzeugte Sinogramminformation. Die Verarbeitungsvorrichtung 100 erhält erste Sinogramminformation und zweite Sinogramminformation. $\alpha$ gibt beispielsweise einen Winkel oder einen Winkelschritt an mit dem Strahler 106 und Detektor 104 zum Objekt stehen. Die Verarbeitungsvorrichtung 100 erhält erste Sinogramminformation und zweite Sinogramminformation aus den vom Detektor 104 aufgezeichneten Projektionen. Der Detektor kann ein Zeilendetektor oder ein Flächendetektor sein. Nach dem Erhalten der ersten Sinogramminformation und der zweiten Sinogramminformation, kann die Verarbeitungsvorrichtung eine Lückenfüller-Sinogramminformation erzeugen. Die Lückenfüller-Sinogramminformation kann eine Lücke in einer zweiten Sinogramminformation füllen. Beispielsweise kann eine erste Sinogramminformation einer Information entsprechen, die mit "low-energy" (LE) spektralen Parametern erzeugt wurde. Eine zweite Sinogramminformation kann beispielsweise einer Information entsprechen, die mit "high-energy" (HE) spektralen Parametern erzeugt wurde. Beispielsweise kann einer erste Sinogramminformation alternativ mit HE spektralen Parametern und eine zweite Sinogramminformation mit LE spektralen Parametern erzeugt werden.

Entsprechend stellt der Detektor 104 die erste Sinogramminformation und die zweite Sinogramminformation zur Verfügung, die von der Verarbeitungsvorrichtung zum Erhalten einer Lückenfüller-Sinogramminformation verarbeitet wird.

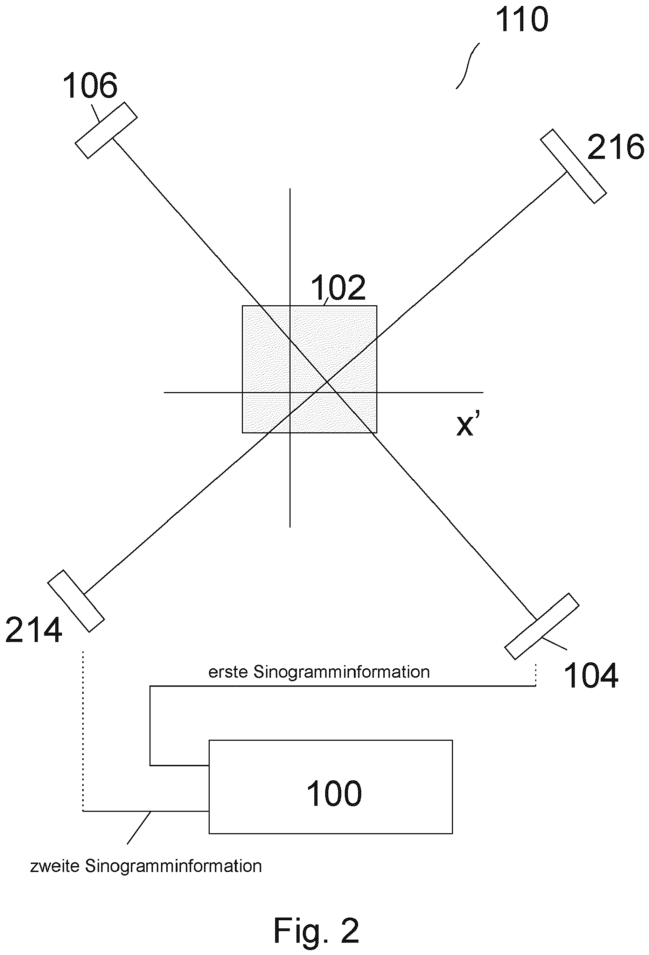

## 2. Verarbeitungsvorrichtung zum Erhalten einer Lückenfüller-Sinogramminformation gemäß Figur 2

Figur 2 unterscheidet sich von Figur 1 darin, dass ein weitere Strahler 216 und ein weiterer Detektor 214 gezeigt sind. Dies ist eine typische Ausführungsform für das im Wesentlichen parallele Aufnehmen von Dual-Energy-CTs. Das Objekt 102 wird zeitgleich von Strahlung mit unterschiedlichen spektralen Parametern durchstrahlt. Eine erste Sinogramminformation wird daher von einem ersten Detektor (hier Detektor 104) und eine zweite Sinogramminformation von einem zweiten Detektor (hier Detektor 214) erzeugt, wobei sich die erzeugt Sinogramminformation der ersten Sinogramminformation und der zweiten Sinogramminformation jeweils auf den gleichen Durchstrahlungswinkel beziehen. Somit kann eine erste Projektion, die einem ersten spektralen Parameter zugeordnet ist und eine zweite Projektion, die einem zweiten spektralen Parameter zugeordnet ist für die im Wesentlichen selbe Projektionsrichtung erhalten werden. In der in Figur 1 dargestellten Ausführungsform wird dies durch denselben Detektor in zwei Durchgängen mit unterschiedlichen spektralen Parametern erhalten.

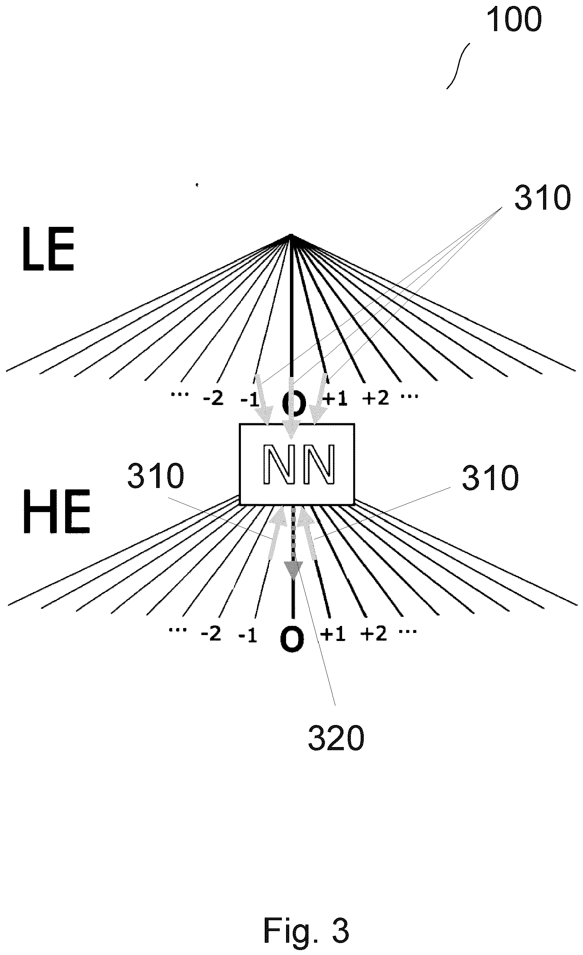

## 3. Verarbeitungsvorrichtung zum Erhalten einer Lückenfüller-Sinogramminformation gemäß Figur 3

Figur 3 zeigt beispielshaft eine Verarbeitungsvorrichtung zum Erhalten einer Lückenfüller-Sinogramminformation 100 mit beispielhaften verschiedenen Projektionen mit Angabe von Winkelschritten und ein neuronales Netz (NN), das konfiguriert ist, um eine Lückenfüller-Sinogramminformation aus einer ersten Sinogramminformation und einer zweiten Sinogramminformation zu erhalten. Hier sind die erste Sinogramminformation und die zweite Sinogramminformation mit LE und HE bezeichnet. Eine Erzeugung von Lückenfüller-Sinogramminformation kann beispielsweise auch mit HE als erster Sinogramminformation und LE als zweiter Sinogramminformation erfolgen. Die in mit vollen Linien dargestellten Pfeile (310) zeigen beispielhaft, dass erste Sinogramminformation (die Pfeile, die von oben in das mit NN bezeichnete neuronale Netz zeigen) und zweite Sinogramminformation (die Pfeile von unten) vom neuronalen Netz (NN) verarbeitet wird zum Erhalten von Lückenfüller-Sinogramminformation, die als gestrichelter Pfeil (320) dargestellt ist. Die Lückenfüller-Sinogramminformation kann beispielsweise die fehlende Information der zweiten Sinogramminformation, die hier bei einem Winkelschritt 0 dargestellt ist, aus den jeweiligen Sinogramminformationen der Winkelschritte -1, +1 und 0 für erste Sinogramminformation (hier LE) erzeugt werden, wie bereits oben in Beispielen dargestellt.

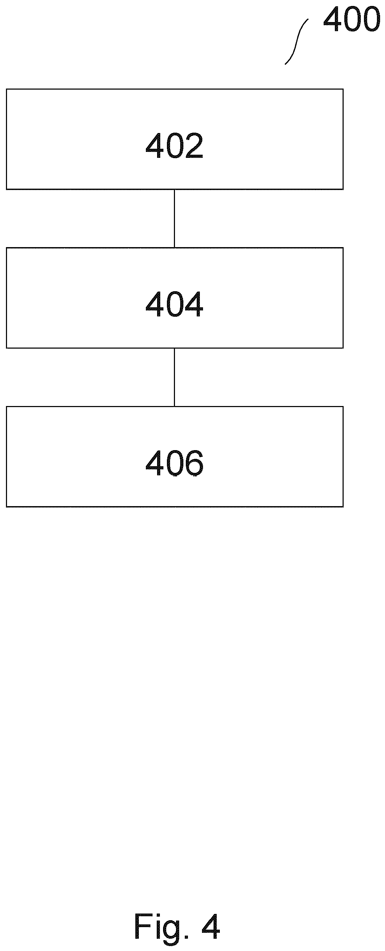

### 4. Verfahren gemäß Figur 4

In der Figur 4 ist beispielhaft ein Verfahren 400 zum Erhalten einer Lückenfüller-Sinogramminformation in einem Blockdiagramm gezeigt. 402 ist dabei das Erhalten einer ersten Sinogramminformation, wobei die erste Sinogramminformation Informationen umfasst, die einem ersten spektralen Parameter zugeordnet sind. 404 bezieht sich auf das Erhalten einer zweiten Sinogramminformation, wobei die zweite Sinogramminformation Informationen umfasst, die einem zweiten spektralen Parameter zugeordnet sind. Und 406 bezieht sich auf das Ermitteln oder Bestimmen einer Lückenfüller-Sinogramminformation, die eine Lücke in der zweiten Sinogramminformation füllt, basierend auf der ersten Sinogramminformation und der zweiten Sinogramminformation.

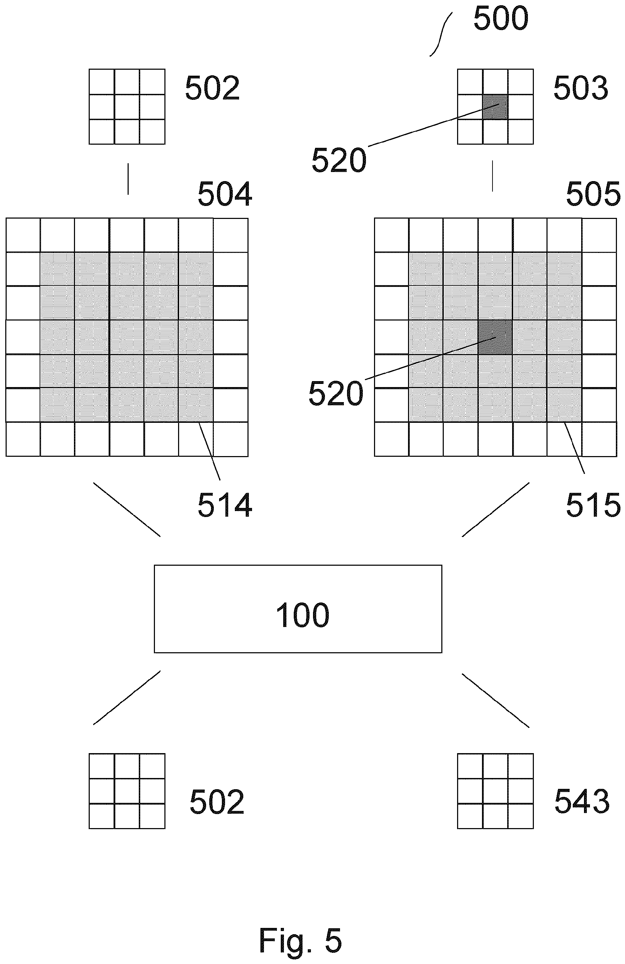

### 5. Verfahren gemäß Figur 5

In der Figur 5 ist beispielhaft ein Verfahren 500 zum Erhalten einer Lückenfüller-Sinogramminformation gezeigt, für das Füllen einer Lücke 520, die hier exemplarisch ein Pixel umfasst, basierend auf einer ersten Sinogramminformation 502 und einer zweiten Sinogramminformation 503. Die Ausdehnung der Pixelgruppen können entlang der räumlichen Achsen in x- und y-Richtung sein. Eine räumliche Ausdehnung der Pixelgruppen kann auch entlang des Winkels sein (beispielsweise je nach Detektor oder Setup). Die Verarbeitungsvorrichtung 100 ist dazu ausgelegt unter Verwendung der ersten Sinogramminformation 502 und der zweiten Sinogramminformation 503 eine Lückenfüller-Sinogramminformation zu erhalten und die Lücke 520 mit der erhaltenen Lückenfüller-Sinogramminformation zu füllen. Anders formuliert, kann die Verarbeitungsvorrichtung eine Lücke mit einer synthetisch erzeugten Lückenfüller-Sinogramminformation füllen. In Figur 5 ist einer erste erhaltene Sinogramminformation 502 dargestellt, und eine zweite Sinogramminformation 503, die sich auf dieselben räumlichen Pixel des untersuchten Objekts beziehen. Die zweite Sinogramminformation 503 weist dabei eine Lücke 520 auf, für die keine Sinogramminformation erhalten wurde. 504 zeigt beispielhaft einen ersten Bereich der ersten Sinogramminformation, die räumlich um die erste erhaltene Sinogramminformation (einschließlich der ersten Sinogramminformation 502) angeordnet ist. Von dem ersten Sinogrammbereich kann ein erster Sinogramm-Rekonstruktionsbereich 514 ausgewählt werden oder pre-determiniert sein, beispielsweise durch eine pre-determinierte Angabe des ersten Sinogramm-Rekonstruktionsbereichs, beispielsweise durch eine pre-determinierte Ausdehnung in eine erste Richtung und eine zweite Richtung (die sich von einer ersten Richtung unterscheidet), wobei die Ausdehnung (beispielsweise pixel-weise) in die erste Richtung größer oder kleiner als die Ausdehnung in die zweite Richtung sein kann. Die im ersten Sinogramm-Rekonstruktionsbereich umfasste Sinogramminformation kann auch als (erste) Sinogramminformationsnachbarn bezeichnet werden, wobei erste Sinogramminformationsnachbarn auch die Sinogramminformation der Lücke umfassen.

Figur 5 zeigt weiter beispielsweise einen zweiten Bereich 505 der zweiten Sinogramminformation, der räumlich um die zweiten erhaltene Sinogramminformation angeordnet ist. Von dem zweiten Sinogrammbereich kann ein Sinogramm-Rekonstruktionsbereich 515 ausgewählt werden oder pre-determiniert sein, beispielsweise durch eine pre-determinierte Angabe des Sinogramm-Rekonstruktionsbereichs, beispielsweise durch eine pre-determinierte Ausdehnung in eine erste Richtung und eine zweite Richtung (die sich von einer ersten Richtung unterscheidet), wobei die Ausdehnung (beispielsweise pixel-weise) in die erste Richtung größer oder kleiner als die Ausdehnung in die zweite Richtung sein kann. Die im zweiten Sinogramm-Rekonstruktionsbereich umfasste Sinogramminformation kann auch als (zweite) Sinogramminformationsnachbarn bezeichnet werden, wobei zweiten Sinogramminformationsnachbarn keine Sinogramminformation für die Lücke umfassen.

Figur 5 zeigt weiter beispielhaft das Ermitteln oder Bestimmen einer Lückenfüller-Sinogramminformation. Die Verarbeitungsvorrichtung 100 erhält die erste Sinogramminformation und die zweite Sinogramminformation in der jeweils der erste Sinogramm-Rekonstruktionsbereich und der zweite Sinogramm-Rekonstruktionsbereich umfasst sein können. Die Verarbeitungsvorrichtung 100 kann beispielsweise durch ein mathematisches Verfahren oder ein neuronales Netz eine Lückenfüller-Sinogramminformation ermitteln. Dies kann beispielsweise mit der ersten Sinogramminformation und der zweiten Sinogramminformation oder den ersten Sinogramminformationsnachbarn und den zweiten Sinogramminformationsnachbarn. Die ermittelte Lückenfüller-Sinogramminformation füllt eine Lücke in der zweiten Sinogramminformation 503, so dass zweite Sinogramminformation entsteht, die keine Lücke aufweist. Diese ist als Ausgangs-Sinogramminformation 543 in Figur 5 dargestellt.

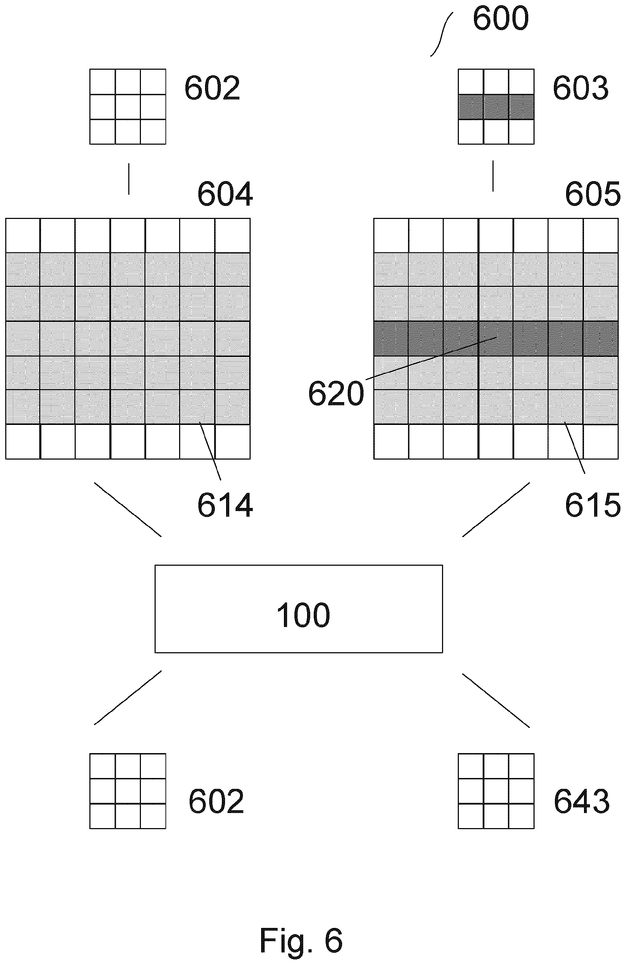

### 6. Verfahren gemäß Figur 6

In der Figur 6 ist beispielhaft ein Verfahren 600 zum Erhalten einer Lückenfüller-Sinogramminformation gezeigt, für das Füllen einer Lücke 620, die hier exemplarisch eine Linie an Pixeln umfasst, basierend auf einer ersten Sinogramminformation 602 und einer zweiten Sinogramminformation 603. Eine Lücke, die einer Linie an Pixeln entspricht, kommt beispielsweise zustande, wenn beim Erhalten einer zweiten Sinogramminformation eine niedrigere Winkelauflösung als beim Erhalten einer ersten Sinogramminformation zugrunde liegt. Eine zweite Sinogramminformation kann damit zumindest eine oder mehrere Lücken, die einer Zeile oder einer Spalte an Pixeln entsprechen, aufweisen.

Für die erhaltenen erste Sinogramminformation und zweite Sinogramminformation kann jeweils aus der räumlichen Umgebung (604, 605) der Lücke 620 ein Bereich (614, 615) ausgewählt werden oder pre-determiniert sein, der für eine Rekonstruktion oder Synthetisierung der Lückenfüller-Sinogramminformation verwendet werden soll, um die Lücke 620 zu füllen. Der ausgewählte Bereich kann in einer Richtung von der Lücke 620 weg einen (beispielsweise pixelweisen) Abstand aufweisen. Der Abstand auf der einen Seite der Lücke und der Abstand auf der anderen Seite der Lücke kann dabei gleich oder unterschiedlich groß sein. Die im ausgewählten oder pre-determinierten Bereich umfasse Sinogramminformation kann auch als Sinogramminformationsnachbarn bezeichnet werden, wobei die Sinogramminformationsnachbarn der ersten Sinogramminformation Sinogramminformation der Lücke umfassen und die Sinogramminformationsnachbarn der zweiten Sinogramminformation keine Sinogramminformation der Lücke umfassen. Die erste Sinogramminformation und die zweite Sinogramminformation werden von der Verarbeitungsvorrichtung 100 zum Ermitteln der Lückenfüller-Sinogramminformation erhalten. Die erhaltene Lückenfüller-Sinogramminformation kann verwendet werden, um eine Lücke 620 in der zweiten Sinogramminformation zu schließen. Die zweite Sinogramminformation, deren Lücke mit Lückenfüller-Sinogramminformation geschlossen wurde kann als Ausgangs-Sinogramminformation 643 bezeichnet werden. Eine Winkelauflösung der zweiten Sinogramminformation kann unter Verwendung von Lückenfüller-Sinogramminformation erhöht werden. Beispielsweise entspricht die Auflösung der Ausgangs-Sinogramminformation der zweiten Sinogramminformation einer Auflösung der ersten Sinogramminformation, beispielsweise einer Winkelauflösung der ersten Sinogramminformation. Die erste Sinogramminformation 602 wird beispielsweise durch das Verfahren nicht verändert.

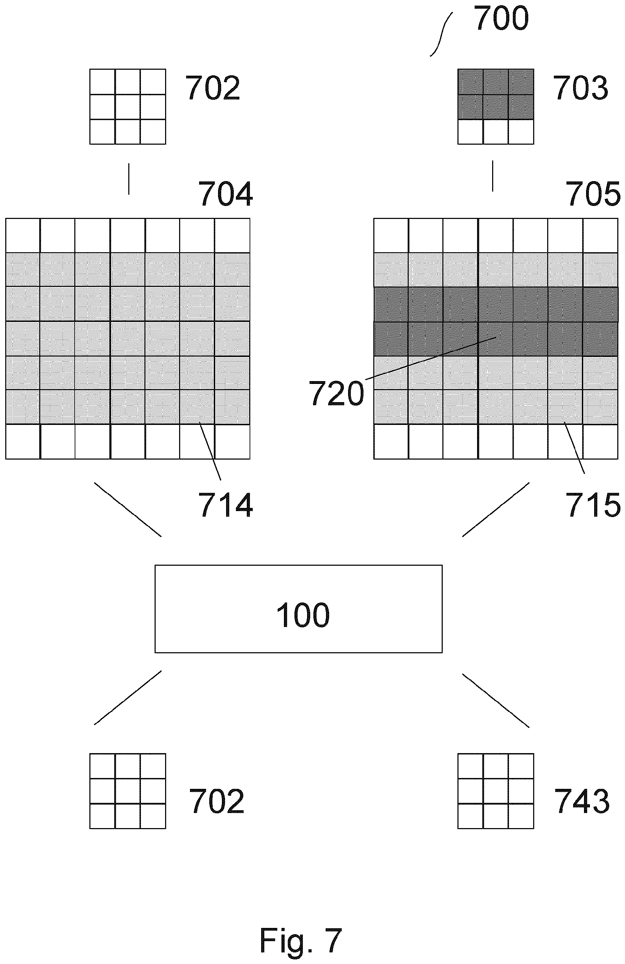

## 7. Verfahren gemäß Figur 7

In der Figur 7 ist beispielhaft ein Verfahren 700 zum Erhalten einer Lückenfüller-Sinogramminformation gezeigt. Eine Lücke 720 in der zweiten Sinogramminformation 703, die hier beispielsweise einem Patch entspricht, kann mit dem Verfahren gefüllt werden. Die erste Sinogramminformation ist mit 702 bezeichnet. Ein Bereich, der räumlich so angeordnet ist, dass er die erste Sinogramminformation umfasst, ist mit 704 bezeichnet. Ein Bereich der räumlich so angeordnet ist, dass er die zweite Sinogramminformation umfasst, ist mit 705 bezeichnet. In dem Bereich 704 und 705 können jeweils Sinogramm-Rektonstruktionsbereiche 714, 715 ausgewählt werden oder pre-determiniert sein. Die ausgewählten oder pre-determinierten Sinogramm-Rektonstruktionsbereiche 714, 715

Die Verarbeitungsvorrichtung 100 erhält die erste Sinogramminformation und die zweite Sinogramminformation in der jeweils der erste Sinogramm-Rekonstruktionsbereich und der zweite Sinogramm-Rekonstruktionsbereich umfasst sein können.

Erste Sinogramminformation und zweite Sinogramminformation können unterschiedliche Größe haben. Sinogramminformation kann beispielsweise dem ganzen Bild, einem Patch, einer Linie oder einem Pixel entsprechen. Ebenso können Sinogramminformationsnachbarn und/oder Sinogramm-Rekonstruktionsbereiche unterschiedliche Größe haben (in Bezug auf die umfassten Pixel, die umfasste Information oder die umfassten Grauwerte). Ein Rekonstruktionsbereich kann größer sein als erste Sinogramminformation, also beispielsweise mehr Pixel umfassen als erste Sinogramminformation.

Beispielsweise kann das Verfahren wiederholt werden, bis alle Lücken mit Lückenfüller-Sinogramminformation geschlossen sind. Beispielsweise kann das Verfahren nur einmal ausgeführt werden, um zumindest im Wesentlichen alle Lücken der zweiten Sinogramminformation zu schließen.

## 8. Weitere Ausführungsbeispiele und Aspekte

Im Folgenden werden weitere Ausführungsbeispiele und Aspekte erläutert. Die weiteren Ausführungsbeispiele und Aspekte können für sich genommen, aber auch in Kombination mit allen anderen hierin offenbarten Ausführungsbeispielen verwendet werden. Die in diesem Abschnitt beschriebenen Ausführungsbeispiele können optional um alle anderen hierin offenbarten Merkmale, Funktionalitäten und Details ergänzt werden, und zwar sowohl einzeln als auch in Kombination. Umgekehrt können alle hierin offenbarten Ausführungsbeispiele optional um alle in diesem Abschnitt erläuterten Merkmale, Funktionalitäten und Details ergänzt werden, und zwar sowohl einzeln als auch in Kombination.

### 8.1. Allgemeiner Lösungsweg

Gemäß einem Aspekt der Erfindung werden zunächst die Projektionsdaten für eine Standard-CT aufgenommen (beispielsweise die erste Sinogramminformation). Anschließen (oder auch zeitlich parallel bzw. zeitlich verzahnt) wird noch ein zweiter CT-Projektionsdatensatz mit angepassten spektralen Parametern (beispielsweise die zweite Sinogramminformation) aufgenommen, allerdings mit einer deutlich reduzierten Abtastung bezüglich des Rotationswinkels (also beispielsweise mit einer im Vergleich zu der ersten Sinogramminformation verringerten Winkelauflösung). Die Aufnahmezeit und die Dosis erhöht sich also beispielsweise nur geringfügig. Zusätzliche Hardware ist (in manchen Fällen) nicht notwendig.

Gemäß einem Aspekt der Erfindung besteht der Ansatz nun darin, beispielsweise die fehlenden HE-Projektionen (oder allgemein die aufgrund der reduzierten Winkelauflösung der zweiten Sinogramminformation fehlenden Sinogrammdaten) beispielsweise aus ähnlichen (vorhandenen) HE-Projektionen, also ähnlichen Winkeln (zum Beispiel aus vorhandenen HE-Projektionen, die winkelmäßig benachbart zu den fehlenden HE-Projektionen sind) sowie beispielsweise den für alle nötigen Winkelschritte vorhandenen LE Projektionen zu generieren.

Es wurde erkannt, dass sich Projektionen aufeinanderfolgender Winkelschritte beispielsweise für gewöhnlich sehr ähnlich sind, da das Winkelinkrement typischerweise deutlich kleiner als 1° ist.

In einem Beispiel zur Veranschaulichung sei (bzw. umfasse) der erste Datensatz (zum Beispiel die erste Sinogramminformation) die low energy (LE) Projektionen (niedrige-Energie Projektionen), der zweite (oder der zweite Datensatz, zum Beispiel die zweite Sinogramminformation) die high energy (HE) Projektionen (hohe-Energie-Projektionen). In der Abtastung der Projektionswinkel sei beim zweiten Datensatz (HE) beispielsweise nur jeder zweite (50% der Anzahl) der Projektionswinkel des ersten Projektionsdatensatzes (LE) ausgeführt (bzw. es ist im zweiten Datensatz beispielsweise eine Sinogrammzeile nur für jeden zweiten Projektionswinkel enthalten), also um 50% reduziert. D.h. im HE-Sinogramm fehlt jede zweite Zeile. (Dies ist allerdings nur ein Beispiel. Im Allgemeinen wird beispielsweise nur jeder n-te Projektionswinkel aufgenommen, dann ist auch nur jede n-te Zeile im entsprechenden Sinogramm, beispielsweise im zweiten Datensatz oder in der zweiten Sinogramminformation, vorhanden)

Für (in diesem Beispiel) 50% der Winkelschritte steht also nur eine LE-Projektion (beispielseweise eine Sinogrammzeile in der ersten Sinogramminformation) zur Verfügung, für die restlichen Projektionswinkel steht sowohl eine LE- als auch eine HE-Projektion (also beispielsweise sowohl eine Sinogrammzeile in der ersten Sinongramminformation also auch eine Sinogrammzeile in der zweiten Sinogramminformation) zur Verfügung.

Damit wird nun beispielsweise ein Tiefes Neuronales Netz (deep neural network, DNN), z.B. in Form von generativen neuronalen Netzen (generative adversarial networks, GAN), trainiert, so dass es aus den Winkelschritten der LE-Projektionen (oder aus den Sinogrammzeilen der LE-Projektionen), für die keine (beispielsweise winkelmäßig zugehörigen) HE-Projektionen vorhanden sind, die (nicht real vorhandenen) HE-Projektionen (also beispielsweise die Lückenfüller-Sinogramminformation) generieren kann.

Mit Blick auf die Sinogramme werden beispielsweise also fehlenden Zeilen im HE-Sinogramm (zum Beispiel als Lückenfüller-Sinogramminformation) erzeugt. Für diese fehlenden Sinogrammzeilen im HE stehen aber beispielsweise im Gegensatz zu [12] und [15] nicht nur die benachbarten Zeilen dieses (HE) Sinogramms (sprich die benachbarten Winkelprojektionen) zur Verfügung (bzw. werden verwendet), sondern auch die Informationen aus dem LE-Sinogramm, die ja die strukturelle Information prinzipiell schon enthält. Daher können die so erzeugten HE-Sinogrammzeilen (HE-Winkelprojektionen) von wesentlich besserer Qualität sein.

Im Folgenden wird ein Beispiel erklärt.

Die in diesem Beispiel zur Verfügungen stehende Information für ein Pixel eines solches Systems wäre also beispielsweise:

- Der Grauwert (zum Beispiel Sinogrammwert) des betreffenden Pixels im LE-Bild (Beispiel Pixel an der x-Koordinate 2000, y-Koordinate 1000, Winkelschritt 180, also [2000, 1000, 180]LE)
- Die Grauwerte (zum Beispiel Sinogrammwerte) der räumlichen Nachbarn in einer definierten Umgebung (z.B. der Größe $(2p+1)\times(2q+1)$) dieses Pixels oder Patches im LE Bild ([2000+/-p, 1000+/-q, 180]LE), also hier im Intervall [2000-p, 2000+p], respektive [1000-p,1000+p].
- Die Grauwerte (zum Beispiel Sinogrammwerte) an der Position des betreffenden Pixels in den benachbarten Winkelschritten und seiner definierten räumlichen Umgebung im HE-Bild ([2000+/-p , 1000+/-q, 180-r]HE und [2000+/-p, 1000+/-q, 180+r]HE)
- Die Grauwerte (zum Beispiel Sinogrammwerte) an der Position des betreffenden Pixels in den benachbarten Winkelschritten und seiner definierten räumlichen Umgebung im LE-Bild ([2000+/-p , 1000+/-q, 180-r]LE und [2000+/-p , 1000+/-q, 180+r]LE). Diese Information der benachbarten Winkelschritte in LE ist nicht zwingend nötig, es können aber beispielsweise die benachbarten Winkelschritte in LE mitgenommen werden. Dies entspricht beispielsweise der in Fig. 3 dargestellten Situation.
- Anstelle eines einzelnen Pixels kann auch eine räumlich zusammenhängende Gruppe von Pixeln ("Patch"), im Extremfall also sogar ein ganzes Bild, betrachtet werden.

Die gewünschte Ausgabe ist:

- der Wert des betreffenden Pixels bzw. Patches im HE-Bild, im Falle eines Einzelpixels hier also [2000, 1000, 180]HE.

In der folgenden detaillierteren Erklärung (beispielsweise in den Abschnitten 8.2 und 8.3) werden beispielhaft zwei Lösungen genauer beschrieben:

8.2. Verwendung eines oder mehrerer faltender neuronaler Netze

Im Folgenden wird ein kurzer Überblick gegeben.

Eine Möglichkeit, diesen (beispielhaften) Ansatz zu realisieren ist die Verwendung von tiefen neuronalen Netzen (bzw. eines tiefen neuronalen Netztes), beispielsweise tiefen Faltungsnetzen (Convolutional Neural Networks, CNNs) (bzw. eines tiefen Faltungsnetzes), erzeugende gegnerische Netzwerke (Generative Adversarial Networks, GANs) (bzw. eines erzeugenden generischen Netztwerks) oder beispielsweise einer U-Net Architektur [17]. Auf diese Weise können (beispielsweise) Pixel für Pixel, Patch für Patch oder Bild für Bild die fehlenden HE-Projektionen (beispielsweise die Lückenfüller-Sinogramminformation) generiert werden bzw. die Lücken im Sinogramm der HE Daten geschlossen werden, wobei die strukturelle Information über das Objekt bereits aus dem LE-Scan (beispielsweise aus der ersten Sinogramminformation) bekannt ist und nur die energiespezifischen Unterschiede generiert werden müssen (oder zumindest näherungsweise generiert werden sollen).

8.2.1 Akquisition der Daten

Im Folgenden wird die Akquisition der Daten beispielhaft beschrieben.

Wie oben beschrieben werden (beispielsweise) zunächst die LE-Projektionen (zum Beispiel die erste Sinogramminformation) für alle Winkelschritte gemessen. Im nächsten Schritt werden (beispielsweise) die HE-Projektionen (beispielsweise die zweite Sinogramminformation) unter Auslassen jedes zweiten Winkelschrittes (also mit halber Winkelauflösung) aufgenommen. Um das Trainieren des neuronalen Netzwerks in dem gewählten Beispiel zuzulassen, sollen (oder müssen) (beispielsweise) einige Projektionen für die fehlenden Winkelschritte aufgenommen werden. Dann liegen genug Daten vor um (beispielsweise) einen Trainingsdatensatz bereitzustellen.

8.2.2 Training des Neuronalen Netzwerks

Im Folgenden wird ein Training des Neuronalen Netzwerks (zum Beispiel eines tiefen Faltungsnetzes oder eines erzeugenden gegnerischen Netzwerks, oder einer U-Net-Architektur bzw. U-Netz-Architektur) beispielhaft beschrieben. In diesem Zusammenhang wird auch auf die Funktionalität des neuronalen Netzes eingegangen.

Es gilt nun die fehlenden HE-Projektionen (im konkreten Beispiel HE 0, das hier beispielsweise anhand Figur 3 erklärt wird; beispielsweise eine HE Projektion mit Winkelschrittindex 0) (zum Beispiel die fehlende zweite Sinogramminformation) aus den gemessenen Daten zu berechnen. Die Projektionen, die am meisten Informationen tragen (bzw. der zu interpolierenden Projektion am ähnlichsten sind), die zu der Berechnung der fehlenden HE-Projektionen (zum Beispiel der fehlenden zweiten Sinogramminformation bzw. der Lückenfüller-Sinogramminformation) nützlich und in manchen Fällen nötig sind, sind beispielsweise deren HE-Nachbarprojektionen (HE +/-1; zum Beispiel HE-Projektionen mit Winkelschrittindices -1 und +1, die winkelmäßig zu der HE-Projektion mit Winkelindex 0 benachbart sind), sowie die LE-Projektion (zum Beispiel eine Projektion der ersten Sinogramminformation) mit der gleichen Winkelposition (LE 0) (die beispielsweise einen gleichen Winkelschrittindex aufweist wie die fehlende HE-Projektion HE0) und deren LE-Nachbarprojektionen (LE +/- 1)(die beispielsweise die gleichen Winkelschrittindizes -1 und +1 aufweisen wie die Projektionen HE-1 und HE+1).

Es bietet sich deshalb an, beispielsweise diese Projektionen (oder auch genau diese Projektionen) als Input (also zum Beispiel als Eingangsinformation oder als Eingangsgrößen) für ein Neuronales Netzwerk herzunehmen, welches als Output (zum Beispiel als Ausgangsgröße) die zu interpolierende Projektion ausgeben soll.

Figur 3 zeigt die verwendeten Input Winkelpositionen bzw. Eingangs-Winkelpositionen (Pfeile 320a,320b,320c,322a,322b) und die Output Winkelprojektion bzw. Ausgangs-Pinkelprojektion (Pfeil 330), die beispielsweise einer Lückenfüller-Sinogramminformation entspricht, für dieses Beispiel.

Es ist dabei beispielsweise möglich jeweils nur einen kleinen Bildausschnitt (zum Beispiel einen Patch) der Projektionen zu betrachten. Durch den geringen Winkelunterschied zwischen benachbarten Projektionen, liegen die Informationen, die zur Interpolation eines Bereichs in der HE-Output Projektion (zum Beispiel in der HE-Ausgangsprojektion oder in der Lückenfüller-Sinogramminformation) nötig sind, an den nahezugleichen Positionen in der entsprechenden LE-Projektion (zum Beispiel in der ersten Sinogramminformation) und beispielsweise sehr nah (zum Beispiel wenige Pixel Abstand) in den benachbarten Projektionen aus HE- und LE-Akquisition (zum Beispiel zweiter Sinogramminformations-Akquisition und erster Sinogramminformations-Akquisition).

Aufgrund der geringen aber vorhanden Verschiebung kann (oder sollte) beispielsweise ein gewisser Sicherheitssaum zwischen der Größe des Input-Patches (zum Beispiel Eingangs-Bereichs bzw. Eingangs-Patches) und des Output-Patches (zum Beispiel Ausgangs-Bereichs bzw. Ausgangs-Patches), wobei beispielsweise (Input > Output), oder wobei beispielsweise der Eingangs-Bereich größer als der Ausgangs-Bereich ist, eingeräumt sein.

Ein einfaches Convolutional Neural Network (CNN bzw. faltendes neuronales Netzwerk) kann bereits diese Aufgabe erfüllen.

Beispielhaft sei hier ein einfaches CNN mit 6 Hidden Layern (verborgenen Schichten) genannt (Größenangaben in Pixeln, x- und y-Koordinate entsprechen der x- und y-Koordinate in der Projektion, die z-Koordinate entspricht dem Winkelschritt der Projektion):

Input Layer bzw. Eingangsschicht (x-dim bzw. x-Dimension: 15 ,y-dim bzw. y-Dimension: 15, z-dim bzw. z-Dimension: 5)

Convolutional Layer (faltende Schicht) 1 (Kernelgröße: 3x3, Filterzahl: 20)

Convolutional Layer (faltende Schicht) 2 (Kernelgröße: 3x3, Filterzahl: 30)

Convolutional Layer (faltende Schicht) 3 (Kernelgröße: 3x3, Filterzahl: 5)

Convolutional Layer (faltende Schicht) 4 (Kernelgröße: 3x3, Filterzahl: 4)

Convolutional Layer (faltende Schicht) 5 (Kernelgröße: 3x3, Filterzahl: 3)

Convolutional Layer (faltende Schicht) 6 (Kernelgröße: 3x3, Filterzahl: 1)

Output Layer bzw. Ausgangsschicht (x-dim bzw. X-Dimension: 3, y-dim bzw. y-Dimension:3, z-dim bzw. z-Dimension: 1)

Das oben skizzierte Netzwerk kann beispielsweise aus 5 (15px x 15px) Patches (bzw. Bereichen) der in Fig. 3 beschriebenen 3 LE- und 2 HE-Input-Projektionen bzw. Eingangs-Projektionen (zum Beispiel aus der ersten Sinogramminformation für drei Winkelschritte und der zweiten Sinogramminformation für zwei Winkelschritte) einen (3px x 3px) HE-Output Patch generieren.

Beispielsweise können auch 3 HE-Input-Projektionen und 2 LE-Input-Projektionen verwendet werden um beispielsweise eine Lücke in den LE-Input-Projektionen zu füllen. Die erste Sinogramminformation kann beispielsweise sowohl eine HE-Projektion als auch, alternativ, eine LE-Projektion sein, und die zweite Sinogramminformation kann beispielsweise sowohl eine HE-Projektion als auch, alternativ, eine LE Projektion sein, sofern beispielsweise die erste Sinogramminformation einem anderen spektralen Parameter zugeordnet ist als die zweite Sinogramminformation.

Dazu (beispielsweise um einen HE-Ausgangsbereich bzw. HE-Output Patch zu generieren, oder um einen LE-Ausgangsbereich oder LE-Output-Patch zu generieren) kann das Netzwerk zunächst trainiert werden.

Zum Training des Netzwerks können als Output-Labels (zum Beispiel als Ausgangs-Markierungen bzw. als Ausgabe bzw. als Trainings-Referenzwerte bzw. Trainings-Soll-Ausgabewerte) beispielsweise (3px x 3px) Patches (bzw. Bereiche) der erfassten HE-Projektionen hergenommen und als Input (zum Beispiel als Eingabe bzw. als Trainings-Eingangswerte) die (15px x 15px) Patches (bzw. Bereiche) aus den oben beispielhaft beschriebenen entsprechenden 3 LE- und 2 HE-Input-Projektionen (bzw. Eingangs-Projektionen bzw. Eingabe-Projektionen).

Vor dem Training kann (optional) ein geringer Teil der Trainings-Patches aus dem Trainings-Datensatz entfernt und als Validierungsdatensatz zur Durchführung des Early Stopping (bzw. des frühen Abbruchs) zum rechtzeitigen Abbruch des Trainings aufbewahrt werden.

Zur Optimierung des Trainingsprozesses können die Input- und Output-Daten (zum Beispiel die Eingangsdaten und die Ausgangsdaten, oder die Eingangs-Sinogramminformation und die (erwartete) Lückenfüller-Sinogramminformation) normalisiert und standardisiert werden.

Diese Skalierung kann beispielsweise beim Anwenden (Inference) des Neuronalen Netzwerks beachtet werden.

Zum Trainieren des Neuronalen Netzwerks werden die Trainings-Daten beispielswese (optional) augmentiert und es können (optional) weitere Techniken zur Verhinderung von Overfitting (Überanpassung) angewendet werden (zum Beispiel Regularization bzw. Regularisierung, und/oder Dropout bzw. Herausnahme, und/oder Noise bzw. Rauschen, und/oder Early Stopping bzw. frühes Abbrechen).

Die beim Trainieren des Neuronalen Netzwerks optimierten Parameter, können beispielsweise entweder zufällig oder durch Parameter aus einem vor-trainierten Netzwerk mit der gleichen Architektur, welches erfolgreich zur Lösung eines ähnlichen Problems trainiert wurde, initialisiert werden (Transfer learning bzw. Transfer-Lernen).

Weitere Details bezüglich einiger hier erwähnter Techniken aus den Bereichen Machine Learning / Deep Learning (Maschinenlernen bzw. tiefes Lernen) sind in der Fachliteratur zu finden.

Im Folgenden wird ein Auffüllen der fehlenden Projektionen beschrieben. Dies kann beispielsweise erfolgen, wenn das neuronale Netzwerk trainiert ist.

8.2.3 Auffüllen der fehlenden Projektionen

Ist das Neuronale Netzwerk trainiert, können schließlich die fehlenden HE-Projektionen erzeugt werden. Die nötigen Input-Daten (LE- und HE-Projektionen), die beispielsweise in der Akquisition erzeugt wurden, werden beispielsweise in die entsprechenden 5er Sets von (15px x 15px) Patches (bzw. Bereichen) zerlegt, sodass damit die (3px x 3px) Output-Patches beispielsweise mit dem neuronalen Netzwerk erzeugt werden können. Um schließlich die Projektionen zu erlangen, können diese aus den (3px x 3px) Patches zusammengesetzt werden. Dabei ist zu beachten, dass bei manchen Architekturen des Netzwerks, und/oder bei mancher Wahl des Inputs und des Outputs (erste und zweite Sinogramminformation und Lückenfüller-Sinogramminformation), beispielsweise durch die Architektur des Netzwerks, wie sie im Beispiel angegeben ist, , beispielsweise Informationen am Rand der Projektionen nicht interpoliert werden können. Sind diese Informationen für die Rekonstruktion des CTs nicht von Relevanz, so können diese beispielsweise durch Pixel mit einem wählbaren Grauwert aufgefüllt werden. Sollen jedoch auch diese Pixel interpoliert werden so können die Input-Projektionen (zum Beispiel die erste Sinogramminformation und die zweite Sinogramminformation) beispielsweise mit schwarzen Pixeln erweitert werden, bis die durch die erzeugten Patches rekonstruierbare HE-Projektion (zum Beispiel die Lückenfüller-Sinogramminformation) der erwarteten Größe entspricht.

Gemäß Ausführungsformen können beim Zusammensetzen der Interpolation überlappende Patches (Pixelbereiche) verwendet werden, welche beispielsweise durch gewichtetes Mitteln das endgültige HE-Bild (z.B. die zweite Sinogramminformation als Ausgangs-Sinogramminformation) an der fehlenden Winkelposition ergeben.

In diesem Beispiel, dessen Merkmale auch allgemeingültig mit Ausführungsformen hierein kombiniert werden können, wurde angenommen, dass nur die Hälfte der Projektionen des HE-Datensatzes (z.B. der zweiten Sinogramminformation) ausgelassen wurde. Analog können jedoch auch % aller Projektionen ausgelassen werden. Dann kann ein neuronales Netzwerk konstruiert oder angepasst werden, welches jeweils die 3 fehlenden HE-Projektionen (zum Beispiel zweite Sinogramminformation bzw. Lücken der zweiten Sinogramminformation) zwischen zwei akquirierten HE-Projektionen (zweite Sinogramminformation) interpoliert. Als Input-Daten (Eingangsdaten) können dann beispielsweise die 3 entsprechenden LE-Projektionen (zum Beispiel der ersten Sinogramminformation) plus deren direkte Nachbarn (zum Beispiel Sinogramminformationsnachbarn der ersten Sinogramminformation) und die direkten HE-Nachbarn (zum Beispiel Sinogramminformationsnachbarn der zweiten Sinogramminformation) der zu erzeugenden HE-Projektionen (z.B. der Lücken in der zweiten Sinogramminformation bzw. der Lückenfüller-Sinogramminformation) herangezogen werden.

Es werden also beispielsweise mit den Informationen von 7 Projektionen, hier beispielsweise (5xLE + 2xHE) 3 HE-Projektionen (zum Beispiel Lückenfüller-Sinogramminformation) generiert. Daraus können Qualitätseinbußen im Vergleich mit der im obigen Beispiel beschriebenen Interpolation (3xLE + 2xHE -> 1xHE) folgen. Es liegen aber auch weniger HE Projektionen vor (25% statt 50% der LE-Projektionen).

Je nach Einsatz können diese aber im Rahmen der gewünschten und/oder erforderlichen Qualität liegen und gegenüber der (3xLE + 2xHE -> 1xHE) einen Zeitvorteil in der Akquisition und dem Erzeugen der Lückenfüller-Sinogramminformation haben.

Die hier beispielhaft beschriebene Architektur des CNN ist eine primitive Realisation des Lösungswegs. In der praktischen Umsetzung hat sich ein auf der U-Net-Architektur basierendes neuronales Netzwerk als akkurat erwiesen. Allerdings ist es ebenfalls möglich, andere Netzwerkarchitekturen eines neuronalen Netzwerks zu verwenden, die in manchen Fällen die Effizienz des hier beschriebenen U-Nets übertreffen können. Diese können beispielsweise adaptiert werden, um erfindungsgemäß Lückenfüller-Sinogramminformation zu erhalten.

Im Folgenden werden einige Schlussfolgerungen dargelegt.

Ausführungsbeispiele gemäß der vorliegenden Erfindung beschränken sich nicht auf die Verwendung eines spezifischen Neuronalen Netzwerks und/oder einer Netzwerkarchitektur.

Ausführungsbeispiele der vorliegenden Erfindung betreffen vielmehr ein neues Erzeugungsprinzip für DE-CTs, welches sich durch folgende Schritte zusammenfassen lässt:

1. Akquisition von LE-Projektionen für alle Winkelpositionen (beispielsweise einen Satzes der Winkelpositionen)

2. Akquisition von HE-Projektionen für einen Bruchteil (oder beispielsweise eine echte Teilmenge) der Winkelprojektionen (beispielsweise des Satzes von Winkelpositionen)

3. Training eines neuronalen Netzwerks zur Interpolation der fehlenden Winkelpositionen anhand der aufgenommenen LE- und HE-Daten

4. Auffüllen der fehlenden HE-Projektionen durch Anwendung des trainierten neuronalen Netzwerks.

8.3 Polynomialer Fit von LE/HE Bildbereichen

Bei diesem Lösungsansatz werden, wie beim oben (zum Beispiel in Abschnitt 8.2) beschriebenen Ansatz mit CNNs, beispielsweise fünf Projektionen als Eingangsdaten verwendet: Die zu der zu erzeugenden HE-Projektion (also beispielsweise der Lücke in der zweiten Sinogramminformation) benachbarten HE und LE Projektionen (je 2, zusammen 4) (beispielsweise Sinogrammwerte oder Sinogrammzeilen der ersten Sinogramminformation und der zweiten Sinogramminformation) sowie die zur zu erzeugenden HE-Projektion gehörende LE-Projektion (beispielsweise ein Sinogrammwert oder eine Sinogrammzeile der ersten Sinogramminformation).

Die zu erzeugende HE-Projektion wird beispielsweise in Patches (zum Beispiel Bereiche) unterteilt (zum Beispiel aneinander angrenzende Bildstücke der Projektion mit fester Größe), im Beispiel 4x4 Pixel.

Die zugehörigen Patches in den Eingangsdaten werden beispielsweise um eine gewisse Anzahl an Pixeln größer gewählt, im Beispiel nach links und rechts, oben und unten um je 2 Pixel.

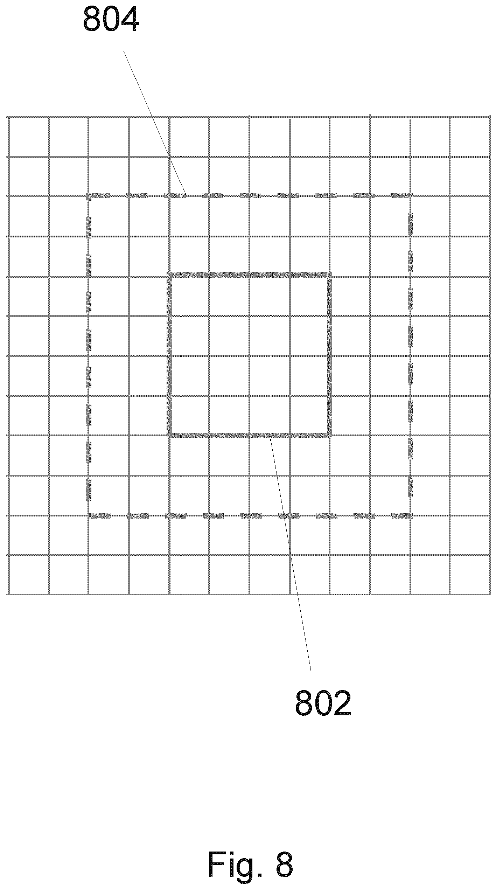

Figur 8 zeigt beispielsweise ein 4x4 Patch für den Output bzw. für die Ausgabe (durchgezogene Linie 802) mit zugehörigem 8x8 Patch der Input-Daten bzw. Eingangsdaten (gestrichelte Linie 804) dieses Winkelschritts.

In einem nächsten Schritt werden beispielsweise die Pixelwerte aus den Eingangs-HE-Patches über den zugehörigen Pixelwerten aus den entsprechenden Eingangs-LE-Patches aufgetragen (bzw. ausgewertet). Dadurch erhält man ein Diagramm (oder eine entsprechende Datenstruktur), in dem ein Punkt von einem Paar aus (beispielsweise zusammengehörigen, beispielsweise gleichen Winkelwerten und Ortskoordinaten zugeordneten) Pixelwerten von LE und HE (an der gleichen Position im gleichen Winkelschritt) definiert wird.

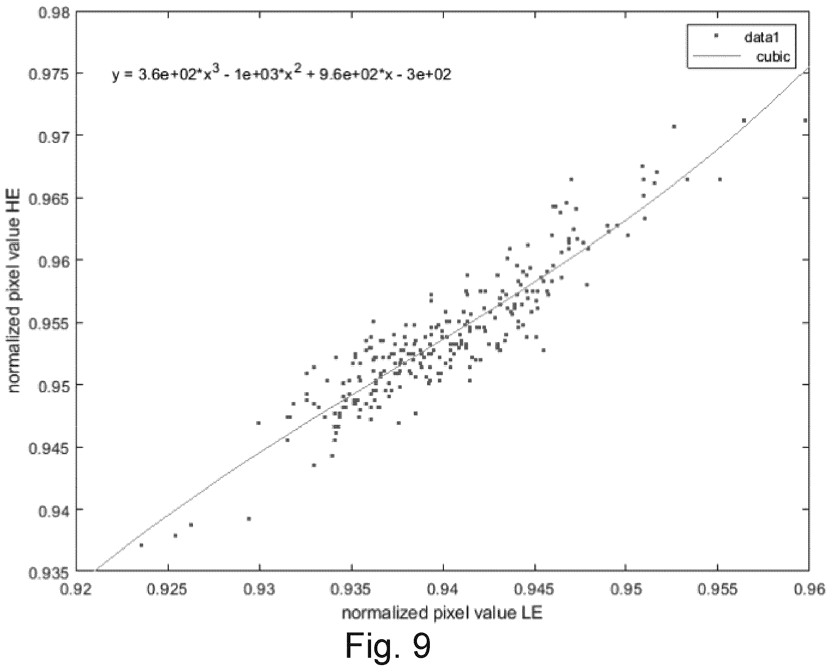

In diese Verteilung wird nun beispielsweise ein Polynom, beispielsweise 3. Grades, gefittet (siehe beispielsweise Figur 9). Mit der so entstandenen Abbildungsvorschrift (die beispielsweise durch die Fitting-Parameter definiert wird, und dem gefitteten Polynom entsprechen kann) von LE-Pixelwerten zu HE-Pixelwerten können nun die Pixelwerte des (kleineren) Output-Patches aus den entsprechenden Pixelwerten des LE-Patches der zugehörigen LE-Winkelposition berechnet werden. Dieser Schritt, inklusive des polynomialen Fits, wird nun für alle Patches der zu erzeugenden HE-Projektion und für alle zu erzeugenden HE-Projektionen durchgeführt.

Im Folgenden wird ein weiteres Beispiel mit HE Projektionen (z.B. Projektionen mit hoher Energie bzw. hoher Beschleunigungsspannung) als zweiter Sinogramminformation und LE Projektionen (z.B. Projektionen mit vergleichsweise niedrigerer Energie bzw. vergleichsweise niedrigerer Beschleunigungsspannung) als erster Sinogramminformation ausgeführt. Dies ist lediglich zur Veranschaulichung gedacht und es ist selbstverständlich, dass HE Projektionen auch als erste Sinogramminformation und LE Projektionen als zweite Sinogramminformation vorliegen können.

In diesem Beispiel werden fünf Eingangsdaten verwendet: Die zu der zu erzeugenden HE-Projektion (z.B. winkelmäßig) benachbarten HE und LE Projektionen (je 2, zusammen 4) sowie die zu der zu erzeugenden HE-Projektion gehörende LE-Projektion (beispielsweise mit gleichem Winkelwert wie die synthetisch zu erzeugende HE Projektion). Dieses Beispiel zeigt das Verfahren an einer spezifischen (einzelnen) Lücke der zweiten Sinogramminformation, wobei die zweite Sinogramminformation, wie im Folgenden beschrieben wird, weitere Lücken umfasst oder beispielsweise umfassen kann. Das beispielhaft beschriebene Verfahren kann dann auf die weiteren Lücken in der zweiten Sinogramminformation angewendet werden, bzw. für die weiteren Lücken wiederholt werden. Die zu erzeugende HE-Projektion (beispielsweise Lückenfüller-Sinogramminformation, die in diesem Beispiel für HE Projektionen als zweite Sinogramminformation erzeugt werden soll) wird beispielsweise in Patches unterteilt (aneinander angrenzende Bildstücke der Projektion mit fester Größe) im Beispiel 4x4 Pixel 802. Die zugehörigen Patches in den Eingangsdaten werden um eine gewisse Anzahl an Pixeln größer gewählt, im Beispiel nach links und rechts, oben und unten um beispielsweise 2 Pixel (entsprechend einer Koordinate in x und y Richtung). Figur 8 zeigt beispielsweise ein 4x4 Patch 802 (durchgezogene Linie) für den zu erhaltenen Output mit zugehörigem beispielhaften 8x8 Patch 804 der Input-Daten (gestrichelte Linie) dieses Winkelschritts.

In einem nächsten Schritt können die Pixelwerte aus den Eingangs-HE-Patches über den zugehörigen Pixelwerten aus den entsprechenden Eingangs-LE-Patches aufgetragen werden. Dadurch erhält man ein Diagramm in dem ein Punkt von einem Paar aus Pixelwerten von LE und HE (an der gleichen Position im gleichen Winkelschritt) definiert wird.

In diese Verteilung wird nun ein Polynom, beispielsweise 3. Grades, gefittet. Mit der so entstandenen Abbildungsvorschrift von LE-Pixelwerten zu HE-Pixelwerten können nun die Pixelwerte des (kleineren) Output-Patches aus den entsprechenden Pixelwerten des LE-Patches der zugehörigen LE-Winkelposition berechnet werden. Dieser Schritt, inklusive des polynomialen Fits, wird nun für alle Patches der zu erzeugenden HE-Projektion und für alle zu erzeugenden HE-Projektionen durchgeführt. So kann beispielsweise eine Lückenfüller-Sinogramminformation unter Verwendung einer mathematischen Methode synthetisiert (erhalten) werden.

In der Figur 9 ist beispielsweise eine Verteilung von normalisierten Pixelwerten für HE und LE Pixelwerte aufgetragen. Diese ist hier beispielsweise mit einem Polynom dritten Grades gefittet. Dieser Fit kann beispielsweise als Abbildungsvorschrift von LE Pixelwerten zu HE Pixelwerten verwendet werden.

Pixelwerte aus den Eingangs-HE-Patches können beispielsweise über den zugehörigen Pixelwerten aus den entsprechenden Eingangs-LE-Patches aufgetragen werden. Dadurch erhält man ein Diagramm in dem ein Punkt von einem Paar aus Pixelwerten von LE und HE (an der gleichen Position im gleichen Winkelschritt) definiert wird.

In diese Verteilung wird nun ein Polynom, beispielsweise 3. Grades, gefittet (siehe Figur 9). Mit der so entstandenen Abbildungsvorschrift von LE-Pixelwerten zu HE-Pixelwerten (hier als Beispiel für erste Sinogramminformation und zweite Sinogramminformation) können nun die Pixelwerte des (kleineren) Output-Patches (bzw. Ausgabe-Bereichs) aus den entsprechenden Pixelwerten des LE-Patches der zugehörigen LE-Winkelposition berechnet werden. Dieser Schritt, inklusive des polynomialen Fits, wird nun für alle Patches der zu erzeugenden HE-Projektion und für alle zu erzeugenden HE-Projektionen durchgeführt. HE Pixelwerte und LE Pixelwerte können in anderen Fällen auch der jeweils anderen Sinogramminformation zugehören. Beispielsweise würde dann eine Abbildungsvorschrift von HE-Pixelwerten zu LE-Pixelwerten ermittelt werden.

Es versteht sich, dass Modifikationen und Variationen der hierin beschriebenen Ausführungsformen und Details für andere Fachleute offensichtlich sind. Ausführungsformen sind daher, auch wenn nicht explizit ausgeführt, miteinander kombinierbar. Es ist daher die Absicht, nur durch den Umfang der bevorstehenden Patentansprüche und nicht durch die spezifischen Details, die durch die Beschreibung und Erläuterung der Ausführungsformen hier vorgestellt begrenzt werden.

## 9. Vorteile

Im Folgenden werden einige mögliche Vorteile, die durch Ausführungsbeispiele gemäß der Erfindung erreicht werden können, noch einmal kurz zusammengefasst.

Die hierin offenbarten Merkmale bzw. die hierin offenbarte Lösung ermöglicht beispielsweise, die Vorteile von DE-CT (z.B. Zwei-Energie-Computertomographie) auszunutzen, beispielsweise ohne die - normalerweise doppelte - Messzeit in Kauf nehmen zu müssen oder ohne erhöhten Hardwareeinsatz mit beispielsweise zweiter Röhre und zweitem Detektor. Der Vorteil besteht beispielsweise in der Kostenersparnis und/oder Dosisminimierung.

## 10. Anwendungsgebiete

Im Folgenden werden einige Anwendungsgebiete von Ausführungsbeispielen gemäß der vorliegenden Erfindung erläutert.

Beispielsweise können Ausführungsbeispiele gemäß der vorliegenden Erfindung bei der Dual-Energy-CT in der Medizin, vor allem zur Reduktion der Patientendosis und/oder Erhöhung der Scangeschwindigkeit bzw. der Verringerung der Komplexität des Gesamtsystems und der Kosten, eingesetzt werden.

Beispielsweise können Ausführungsbeispiele gemäß der vorliegenden Erfindung in Dual-Energy -CT in der technischen und industriellen CT zur Reduktion der Scanzeit bzw. des apparativen Aufwands eingesetzt werden. Die Verwendung von Dual-Energy CT kann die Qualität von CT Scans erhöhen, das "Umgehen" der zusätzlichen Messzeit führt beispielsweise zu einer Kostenreduktion (in Form einer Messzeitreduktion). Analog zur Medizintechnik wird ebenfalls die benötigte Dosis reduziert.

Andere Anwendungsbereiche sind aber ebenso denkbar.

## 11. Implementierungsalternativen

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar. Einige oder alle der Verfahrensschritte können durch einen Hardware-Apparat (oder unter Verwendung eines Hardware-Apparats), wie zum Beispiel einen Mikroprozessor, einen programmierbaren Computer oder eine elektronische Schaltung ausgeführt werden. Bei einigen Ausführungsbeispielen können einige oder mehrere der wichtigsten Verfahrensschritte durch einen solchen Apparat ausgeführt werden.

Je nach bestimmten Implementierungsanforderungen können Ausführungsbeispiele der Erfindung in Hardware oder in Software implementiert sein. Die Implementierung kann unter Verwendung eines digitalen Speichermediums, beispielsweise einer Floppy-Disk, einer DVD, einer Blu-ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einem programmierbaren Computersystem derart zusammenwirken können oder zusammenwirken, dass das jeweilige Verfahren durchgeführt wird. Deshalb kann das digitale Speichermedium computerlesbar sein.

Manche Ausführungsbeispiele gemäß der Erfindung umfassen also einen Datenträger, der elektronisch lesbare Steuersignale aufweist, die in der Lage sind, mit einem programmierbaren Computersystem derart zusammenzuwirken, dass eines der hierin beschriebenen Verfahren durchgeführt wird.

Allgemein können Ausführungsbeispiele der vorliegenden Erfindung als Computerprogrammprodukt mit einem Programmcode implementiert sein, wobei der Programmcode dahin gehend wirksam ist, eines der Verfahren durchzuführen, wenn das Computerprogrammprodukt auf einem Computer abläuft.

Der Programmcode kann beispielsweise auch auf einem maschinenlesbaren Träger gespeichert sein.

Andere Ausführungsbeispiele umfassen das Computerprogramm zum Durchführen eines der hierin beschriebenen Verfahren, wobei das Computerprogramm auf einem maschinenlesbaren Träger gespeichert ist.

Mit anderen Worten ist ein Ausführungsbeispiel des erfindungsgemäßen Verfahrens somit ein Computerprogramm, das einen Programmcode zum Durchführen eines der hierin beschriebenen Verfahren aufweist, wenn das Computerprogramm auf einem Computer abläuft.

Ein weiteres Ausführungsbeispiel der erfindungsgemäßen Verfahren ist somit ein Datenträger (oder ein digitales Speichermedium oder ein computerlesbares Medium), auf dem das Computerprogramm zum Durchführen eines der hierin beschriebenen Verfahren aufgezeichnet ist. Der Datenträger, das digitale Speichermedium oder das computerlesbare Medium sind typischerweise gegenständlich und/oder nicht-vergänglich bzw. nicht-vorübergehend.

Ein weiteres Ausführungsbeispiel des erfindungsgemäßen Verfahrens ist somit ein Datenstrom oder eine Sequenz von Signalen, der bzw. die das Computerprogramm zum Durchführen eines der hierin beschriebenen Verfahren darstellt bzw. darstellen. Der Datenstrom oder die Sequenz von Signalen kann bzw. können beispielsweise dahin gehend konfiguriert sein, über eine Datenkommunikationsverbindung, beispielsweise über das Internet, transferiert zu werden.

Ein weiteres Ausführungsbeispiel umfasst eine Verarbeitungseinrichtung, beispielsweise einen Computer oder ein programmierbares Logikbauelement, die dahin gehend konfiguriert oder angepasst ist, eines der hierin beschriebenen Verfahren durchzuführen.

Ein weiteres Ausführungsbeispiel umfasst einen Computer, auf dem das Computerprogramm zum Durchführen eines der hierin beschriebenen Verfahren installiert ist.

Ein weiteres Ausführungsbeispiel gemäß der Erfindung umfasst eine Vorrichtung oder ein System, die bzw. das ausgelegt ist, um ein Computerprogramm zur Durchführung zumindest eines der hierin beschriebenen Verfahren zu einem Empfänger zu übertragen. Die Übertragung kann beispielsweise elektronisch oder optisch erfolgen. Der Empfänger kann beispielsweise ein Computer, ein Mobilgerät, ein Speichergerät oder eine ähnliche Vorrichtung sein. Die Vorrichtung oder das System kann beispielsweise einen Datei-Server zur Übertragung des Computerprogramms zu dem Empfänger umfassen.

Bei manchen Ausführungsbeispielen kann ein programmierbares Logikbauelement (beispielsweise ein feldprogrammierbares Gatterarray, ein FPGA) dazu verwendet werden, manche oder alle Funktionalitäten der hierin beschriebenen Verfahren durchzuführen. Bei manchen Ausführungsbeispielen kann ein feldprogrammierbares Gatterarray mit einem Mikroprozessor zusammenwirken, um eines der hierin beschriebenen Verfahren durchzuführen. Allgemein werden die Verfahren bei einigen Ausführungsbeispielen seitens einer beliebigen Hardwarevorrichtung durchgeführt. Diese kann eine universell einsetzbare Hardware wie ein Computerprozessor (CPU) sein oder für das Verfahren spezifische Hardware, wie beispielsweise ein ASIC.

Die hierin beschriebenen Vorrichtungen können beispielsweise unter Verwendung eines Hardware-Apparats, oder unter Verwendung eines Computers, oder unter Verwendung einer Kombination eines Hardware-Apparats und eines Computers implementiert werden.

Die hierin beschriebenen Vorrichtungen, oder jedwede Komponenten der hierin beschriebenen Vorrichtungen können zumindest teilweise in Hardware und/oder in Software (Computerprogramm) implementiert sein.

Die hierin beschriebenen Verfahren können beispielsweise unter Verwendung eines Hardware-Apparats, oder unter Verwendung eines Computers, oder unter Verwendung einer Kombination eines Hardware-Apparats und eines Computers implementiert werden.

Die hierin beschriebenen Verfahren, oder jedwede Komponenten der hierin beschriebenen Verfahren können zumindest teilweise durch Hardware und/oder durch Software ausgeführt werden.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

**Literaturverzeichnis**

[1] Herman, G. T. (2009). Fundamentals of computerized tomography: image reconstruction from projections. Springer Science & Business Media.

[2] Feldkamp, L. A., Davis, L. C., & Kress, J. W. (1984). Practical cone-beam algorithm. Josa a, 1(6), 612-619.

[3] Mathews, J. D., Forsythe, A. V., Brady, Z., Butler, M. W., Goergen, S. K., Byrnes, G. B., ... & McGale, P. (2013). Cancer risk in 680 000 people exposed to computed tomography scans in childhood or adolescence: data linkage study of 11 million Australians. Bmj, 346.

[4] Coleman, A. J., & Sinclair, M. (1985). A beam-hardening correction using dual-energy computed tomography. Physics in Medicine & Biology, 30(11), 1251.

[5] Kuchenbecker, S., Faby, S., Sawall, S., Lell, M., & Kachelrieß, M. (2015). Dual energy CT: How well can pseudo-monochromatic imaging reduce metal artifacts?. Medical physics, 42(2), 1023-1036.

[6] Dremel, K., Fuchs, T., Firsching, M., & Hanke, R. (2014). Beam hardening correction in x-ray computed tomography: a comparison of two iterative model-based reconstruction methods 11th European Conf. Non-Destructive Testing (Prague, Czech Republic,).

[7] Alvarez, R. E., & Macovski, A. (1976). Energy-selective reconstructions in x-ray computerised tomography. Physics in Medicine & Biology, 21(5), 733.

[8] Johnson, T. R., Krauss, B., Sedlmair, M., Grasruck, M., Bruder, H., Morhard, D.,... & Flohr, T. (2007). Material differentiation by dual energy CT: initial experience. European radiology, 17(6), 1510-1517.

[9] Patino, M., Prochowski, A., Agrawal, M. D., Simeone, F. J., Gupta, R., Hahn, P. F., & Sahani, D. V. (2016). Material separation using dual-energy CT: current and emerging applications. Radiographics, 36(4), 1087-1105.

[10] Poirot, M. G., Bergmans, R. H., Thomson, B. R., Jolink, F. C., Moum, S. J., Gonzalez, R. G., ... & Gupta, R. (2019). Physics-informed Deep Learning for Dual-energy computed tomography image processing. Scientific reports, 9(1), 1-9.

[11] Zhou, B., Lin, X., Eck, B., Hou, J., & Wilson, D. (2018, December). Generation of virtual dual energy images from standard single-shot radiographs using multi-scale and conditional adversarial network. In Asian Conference on Computer Vision (pp. 298-313). Springer, Cham.

[12] Thaler, F., Hammernik, K., Payer, C., Urschler, M., & Štern, D. (2018, September). Sparse-view CT reconstruction using wasserstein GANs. In International Workshop on Machine Learning for Medical Image Reconstruction (pp. 75-82). Springer, Cham.

[13] Li, Z., Zhang, W., Wang, L., Cai, A., Liang, N., Yan, B., & Li, L. (2019, May). A sinogram inpainting method based on generative adversarial network for limited-angle computed tomography. In 15th International Meeting on Fully Three-Dimensional Image Reconstruction in Radiology and Nuclear Medicine (Vol. 11072, p. 1107220). International Society for Optics and Photonics.

[14] Zhang, R., Zhou, J., Yu, Z., & Nemoto, T. (2020, March). A cascaded deep-learning reconstruction method for sparse-view kV-switching dual-energy CT. In Medical Imaging 2020: Physics of Medical Imaging (Vol. 11312, p. 1131223). International Society for Optics and Photonics.

[15] Ghani, M. U., & Karl, W. C. (2018, June). Deep learning-based sinogram completion for low-dose CT. In 2018 IEEE 13th Image, Video, and Multidimensional Signal Processing Workshop (IVMSP) (pp. 1-5). IEEE.

[16] Clark, D. P., Schwartz, F. R., Marin, D., Ramirez-Giraldo, J. C., & Badea, C. T. (2020). Deep learning based spectral extrapolation for dual-source, dual-energy x-ray computed tomography. Medical Physics.

[17] Ronneberger, O., Fischer, P., & Brox, T. (2015, October). U-net: Convolutional networks for biomedical image segmentation. In International Conference on Medical image computing and computer-assisted intervention (pp. 234-241). Springer, Cham.

# EP 4 057 229 A1

**Patentansprüche**

1. Verarbeitungsvorrichtung (100) zum Erhalten einer Lückenfüller-Sinogramminformation
   wobei die Verarbeitungsvorrichtung (100) ausgelegt ist, um eine erste Sinogramminformation, die einem ersten spektralen Parameter zugeordnet ist, und
   eine zweite Sinogramminformation, die einem zweiten spektralen Parameter zugeordnet ist, zu erhalten, und
   wobei die Verarbeitungsvorrichtung (100) ausgelegt ist, um die Lückenfüller-Sinogramminformation, die dem zweiten spektralen Parameter zugeordnet ist, und
   die die eine Lücke (520, 620, 720) in der zweiten Sinogramminformation füllt, basierend auf der ersten Sinogramminformation und der zweiten Sinogramminformation zu erhalten.

2. Die Verarbeitungsvorrichtung (100) nach Anspruch 1,
   wobei die Lücke (520, 620, 720) einem oder mehreren Pixeln entspricht.

3. Die Verarbeitungsvorrichtung (100) nach einem der Ansprüche 1 oder 2,
   wobei die Verarbeitungsvorrichtung (100) ausgelegt ist, um die Lückenfüller-Sinogramminformation zu erhalten, um
   eine auflösungs-erhöhte Version der zweiten Sinogramminformation zu erhalten.

4. Die Verarbeitungsvorrichtung (100) nach einem der Ansprüche 1 bis 3,
   wobei die erste Sinogramminformation eine erste Winkelauflösung aufweist,
   wobei die zweite Sinogramminformation eine zweite Winkelauflösung aufweist, die kleiner als die erste Winkelauflösung ist, und
   wobei die Verarbeitungsvorrichtung (100) ausgelegt ist, um die Lückenfüller-Sinogramminformation für einen Winkelwert zu erhalten, der zwischen zwei Winkelwerten der zweiten Sinogramminformation liegt.

5. Die Verarbeitungsvorrichtung (100) nach einem der Ansprüche 1 bis 4,
   wobei die zweite Sinogramminformation zum Erhalten der Lückenfüller-Sinogramminformation Sinogramminformation ist, die Sinogramminformationsnachbarn der zweiten Sinogramminformation zur Lücke (520, 620, 720) umfasst.

6. Die Verarbeitungsvorrichtung (100) nach einem der Ansprüche 1 bis 5,
   wobei die Verarbeitungsvorrichtung (100) ausgelegt ist, um die Lückenfüller-Sinogramminformation basierend auf einem oder mehreren der Lücke benachbarten Sinogrammwerten der zweiten Sinogramminformation und zusätzlich einem oder mehreren der Lücke benachbarten Sinogrammwerten der ersten Sinogramminformation und/oder einem oder mehreren der Lücke zugeordneten Sinogrammwerten der ersten Sinogramminformation zu erhalten.

7. Die Verarbeitungsvorrichtung (100) nach einem der Ansprüche 1 bis 6,
   wobei die erste Sinogramminformation zum Erhalten der Lückenfüller-Sinogramminformation Sinogramminformation ist, die Sinogramminformationsnachbarn der ersten Sinogramminformation zur Lücke und erste Sinogramminformation der Lücke umfasst.

8. Die Verarbeitungsvorrichtung (100) nach einem der Ansprüche 1 bis 7,
   wobei die Verarbeitungsvorrichtung (100) dazu ausgelegt ist, die erste und die zweite Sinogramminformation gleichzeitig mit einem Scan oder sequenziell mit zwei Scans zu erhalten.

9. Die Verarbeitungsvorrichtung (100) nach einem der Ansprüche 1 bis 8,
   wobei die erste Sinogramminformation eine Mehrzahl von Durchstrahlungsbild-Zeilen oder Durchstrahlunsbildern aufweist, die verschiedenen Winkeln zugeordnet sind, und
   wobei die zweite Sinogramminformation eine Mehrzahl von Durchstrahlungsbild-Zeilen oder Durchstrahlungsbildern aufweist, die verschiedenen Winkeln zugeordnet sind, und
   wobei die erste Sinogramminformation und die zweite Sinogramminformation unterschiedlichen Durchleuchtungs-Strahlungsenergien einer Mehr-Energie-Computertomographien zugeordnet sind.

10. Die Verarbeitungsvorrichtung (100) nach einem der Ansprüche 1 bis 10,
    wobei die Verarbeitungsvorrichtung (100) dazu ausgelegt ist, die Lückenfüller-Sinogramminformation unter Verwendung eines neuronalen Netzes zu erhalten.

11. Die Verarbeitungsvorrichtung (100) nach einem der Ansprüche 1 bis 11,
    wobei das neuronale Netz mindestens eines aus Convolutional Neural Network, Generative Adversarial Network und U-Net umfasst.

12. Computertomograph (110) umfassend mindestens eine Verarbeitungsvorrichtung (100) nach einem der Ansprüche 1 bis 11.

13. Verfahren (400, 500, 600, 700) zum Erhalten einer Lückenfüller-Sinogramminformation, umfassend:

Erhalten einer ersten Singramminformation (402), wobei die erste Sinogramminformation Informationen umfasst, die einem ersten spektralen Parameter zugeordnet sind,
Erhalten einer zweiten Sinogramminformation (404), wobei die zweite Sinogramminformation Informationen umfasst, die einem zweiten spektralen Parameter zugeordnet sind,
Ermitteln einer Lückenfüller-Sinogramminformation (406), die eine Lücke (520, 620, 720) in der zweiten Sinogramminformation füllt, basierend auf der ersten Sinogramminformation und der zweiten Sinogramminformation.

14. Das Verfahren (400, 500, 600, 700) nach Anspruch 13,
wobei das Ermitteln der Lückenfüller-Sinogramminformation erste Sinogramminformation und zweite Sinogramminformation umfasst, wobei die erste Sinogramminformation und die zweite Sinogramminformation jeweils räumliche Sinogramminformationsnachbarn zur Lücke (520, 620, 720) der zweiten Sinogramminformation umfassen und die ersten Sinogramminformation erste Sinogramminformation der Lücke (520, 620, 720) umfasst.

15. Das Verfahren (400, 500, 600, 700) nach einem der Ansprüche 13 bis 14,
wobei die erste Sinogramminformation eine erste Winkelauflösung aufweist und
wobei die zweite Sinogramminformation eine zweite Winkelauflösung aufweist, die kleiner ist als die erste Winkelauflösung, und
wobei das Ermitteln der Lückenfüller-Sinogramminformation umfasst,
Füllen der Lücke (520, 620, 720) mit Lückenfüller-Sinogramminformation.

16. Das Verfahren (400, 500, 600, 700) nach einem der Ansprüche 13 bis 15,
wobei räumliche Sinogramminformationsnachbarn Sinogramminformation in einer Umgebung

$$(2p +1) \times (2q +1) \qquad (i)$$

umfassen,
Sinogramminforamtionsnachbarn der ersten Sinogramminformation Sinogramminformationen [mit Koordinaten]

$$([x +/- p, y +/- q, \alpha]) \qquad (ii),$$

und Sinogramminformationsnachbarn der zweiten Sinogramminformation Sinogramminformationen [mit Koordinaten]

$$([x +/- p, y +/- q, \alpha - r]) \text{ und } ([x +/- p, y +/- q, \alpha + r]) \qquad (iii),$$

umfassen, wobei in (i), (ii) und (iii) p eine pixelweise Abweichung in eine erste Richtung x, die senkrecht zu einem Projektionswinkel $\alpha$, q eine pixelweise Abweichung in eine zweite Richtung y, die parallel zu einer Rotationsachse des Projektionswinkels $\alpha$ und zur ersten Richtung x und r eine Winkelabweichung ist.

17. Das Verfahren (400, 500, 600, 700) nach einem der Ansprüche 13 bis 16,
wobei das Verfahren (400, 500, 600, 700) ferner umfasst:

Entfernen von Strahlhärtungsartefakten basierend auf der ersten Sinogramminforamtion, der zweiten Sinogramminformation und der Lückenfüller-Sinogramminformation, und/oder
Ermitteln von Materialinformationen des untersuchten Objekts, basierend auf der ersten Sinogramminforamtion, der zweiten Sinogramminformation und der Lückenfüller-Sinogramminformation.

18. Das Verfahren (400, 500, 600, 700) nach einem der Ansprüche 13 bis 17,

wobei das Verfahren (400, 500, 600, 700) vor dem Ermitteln der Lückenfüller-Sinogramminformation ein Ermitteln einer Registrierung der ersten Sinogramminforamtion auf die zweiten Sinogramminformation und das Durchführen einer Registrierung der ersten Sinogramminformation auf die zweiten Sinogramminformation umfasst.

19. Computerprogramm, umfassend Befehle, die bei der Ausführung des Programms durch einen Computer diesen veranlassen, das Verfahren (400, 500, 600, 700) nach einem der Ansprüche 13 bis 18 auszuführen.

20. Computerlesbares (Speicher)medium, umfassend Befehle, die bei der Ausführung durch einen Computer diesen veranlassen, das Verfahren (400, 500, 600, 700) nach einem der Ansprüche 13 bis 18 auszuführen.

Fig. 1

110

106

216

102

x'

214

erste Sinogramminformation

104

100

zweite Sinogramminformation

Fig. 2

Fig. 3

400

402

404

406

Fig. 4

Fig. 5

Fig. 6

Fig. 7

804

802

Fig. 8

$$y = 3.6e+02^*x^3 - 1e+03^*x^2 + 9.6e+02^*x - 3e+02$$

Fig. 9

Europäisches
Patentamt

European
Patent Office

Office européen
des brevets

# EUROPÄISCHER RECHERCHENBERICHT

Nummer der Anmeldung

EP 21 16 2349

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | CAO WENCHAO ET AL: "Slow triple kVp switching CT with convolutional neural network based sinogram completion and material decomposition", THE 6TH INTERNATIONAL CONFERENCE ON IMAGE FORMATION IN X-RAY COMPUTED TOMOGRAPHY, 3. August 2020 (2020-08-03), XP055828387, Regensburg, Germany * das ganze Dokument * | 1-20 | INV. G06T11/00 |
| X | CAO WENCHAO ET AL: "GAN-based sinogram completion for slow triple kVp switching CT", PROGRESS IN BIOMEDICAL OPTICS AND IMAGING, SPIE - INTERNATIONAL SOCIETY FOR OPTICAL ENGINEERING, BELLINGHAM, WA, US, Bd. 11595, 15. Februar 2021 (2021-02-15), Seiten 1159520-1159520, XP060139633, ISSN: 1605-7422, DOI: 10.1117/12.2580735 ISBN: 978-1-5106-0027-0 * das ganze Dokument * | 1-20 | |
| X A | US 2020/196972 A1 (ZHOU JIAN [US] ET AL) 25. Juni 2020 (2020-06-25) * Abbildungen 9a, 9b, 12 * * Absätze [0041], [0080] * | 1,12,13, 19,20 2-11, 14-18 | RECHERCHIERTE SACHGEBIETE (IPC) G06T |

Der vorliegende Recherchenbericht wurde für alle Patentansprüche erstellt

| Recherchenort | Abschlußdatum der Recherche | Prüfer |
|---|---|---|
| München | 28. Juli 2021 | Werling, Alexander |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 21 16 2349

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

28-07-2021

| Im Recherchenbericht angeführtes Patentdokument | Datum der Veröffentlichung | Mitglied(er) der Patentfamilie | Datum der Veröffentlichung |
|---|---|---|---|
| US 2020196972 A1 | 25-06-2020 | CN 111345835 A<br>JP 2020099662 A<br>US 2020196972 A1 | 30-06-2020<br>02-07-2020<br>25-06-2020 |

Für nähere Einzelheiten zu diesem Anhang : siehe Amtsblatt des Europäischen Patentamts, Nr.12/82

EPO FORM P0461

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **HERMAN, G. T.** Fundamentals of computerized tomography: image reconstruction from projections. Springer Science & Business Media, 2009
- **FELDKAMP, L. A. ; DAVIS, L. C. ; KRESS, J. W.** *Practical cone-beam algorithm. Josa a,* 1984, vol. 1 (6), 612-619
- **MATHEWS, J. D. ; FORSYTHE, A. V. ; BRADY, Z. ; BUTLER, M. W. ; GOERGEN, S. K. ; BYRNES, G. B. ; MCGALE, P.** Cancer risk in 680 000 people exposed to computed tomography scans in childhood or adolescence: data linkage study of 11 million Australians. *Bmj,* 2013, vol. 346
- **COLEMAN, A. J. ; SINCLAIR, M.** A beam-hardening correction using dual-energy computed tomography. *Physics in Medicine & Biology,* 1985, vol. 30 (11), 1251
- **KUCHENBECKER, S. ; FABY, S. ; SAWALL, S. ; LELL, M. ; KACHELRIEß, M.** Dual energy CT: How well can pseudo-monochromatic imaging reduce metal artifacts?. *Medical physics,* 2015, vol. 42 (2), 1023-1036
- **DREMEL, K. ; FUCHS, T. ; FIRSCHING, M. ; HANKE, R.** Beam hardening correction in x-ray computed tomography: a comparison of two iterative model-based reconstruction methods. *11th European Conf. Non-Destructive Testing,* 2014
- **ALVAREZ, R. E. ; MACOVSKI, A.** Energy-selective reconstructions in x-ray computerised tomography. *Physics in Medicine & Biology,* 1976, vol. 21 (5), 733
- **JOHNSON, T. R. ; KRAUSS, B. ; SEDLMAIR, M. ; GRASRUCK, M. ; BRUDER, H. ; MORHARD, D. ; FLOHR, T.** Material differentiation by dual energy CT: initial experience. *European radiology,* 2007, vol. 17 (6), 1510-1517
- **PATINO, M. ; PROCHOWSKI, A. ; AGRAWAL, M. D. ; SIMEONE, F. J. ; GUPTA, R. ; HAHN, P. F. ; SAHANI, D. V.** Material separation using dual-energy CT: current and emerging applications. *Radiographics,* 2016, vol. 36 (4), 1087-1105
- **POIROT, M. G. ; BERGMANS, R. H. ; THOMSON, B. R. ; JOLINK, F. C. ; MOUM, S. J. ; GONZALEZ, R. G. ; GUPTA, R.** Physics-informed Deep Learning for Dual-energy computed tomography image processing. *Scientific reports,* 2019, vol. 9 (1), 1-9
- Generation of virtual dual energy images from standard single-shot radiographs using multi-scale and conditional adversarial network. **ZHOU, B. ; LIN, X. ; ECK, B. ; HOU, J. ; WILSON, D.** Asian Conference on Computer Vision. Springer, Dezember 2018, 298-313
- Sparse-view CT reconstruction using wasserstein GANs. **THALER, F. ; HAMMERNIK, K. ; PAYER, C. ; URSCHLER, M. ; STERN, D.** International Workshop on Machine Learning for Medical Image Reconstruction. Springer, September 2018, 75-82
- A sinogram inpainting method based on generative adversarial network for limited-angle computed tomography. **LI, Z. ; ZHANG, W. ; WANG, L. ; CAI, A. ; LIANG, N. ; YAN, B. ; LI, L.** 15th International Meeting on Fully Three-Dimensional Image Reconstruction in Radiology and Nuclear Medicine. International Society for Optics and Photonics, Mai 2019, vol. 11072, 1107220
- A cascaded deep-learning reconstruction method for sparse-view kV-switching dual-energy CT. **ZHANG, R. ; ZHOU, J. ; YU, Z. ; NEMOTO, T.** Medical Imaging 2020: Physics of Medical Imaging. International Society for Optics and Photonics, Marz 2020, vol. 11312, 1131223
- Deep learning-based sinogram completion for low-dose CT. **GHANI, M. U. ; KARL, W. C.** 2018 IEEE 13th Image, Video, and Multidimensional Signal Processing Workshop (IVMSP). IEEE, Juni 2018, 1-5
- **CLARK, D. P. ; SCHWARTZ, F. R. ; MARIN, D. ; RAMIREZ-GIRALDO, J. C. ; BADEA, C. T.** Deep learning based spectral extrapolation for dual-source, dual-energy x-ray computed tomography. *Medical Physics,* 2020
- U-net: Convolutional networks for biomedical image segmentation. **RONNEBERGER, O. ; FISCHER, P. ; BROX, T.** International Conference on Medical image computing and computer-assisted intervention. Springer, Oktober 2015, 234-241